# EUROPEAN PATENT SPECIFICATION

(11) **EP 2 491 906 B1**
(45) Date of publication and mention of the grant of the patent: **03.09.2014**
(21) Application number: 10824803.0
(22) Date of filing: 07.10.2010
(51) Int. Cl.: A61F 13/15, A61F 13/49, B65H 37/04, B65H 39/14, B65H 35/08

(54) **METHOD AND DEVICE FOR MANUFACTURING COMPOSITE OF CONTINUOUS SHEETS FOR ABSORPTIVE ARTICLE**
VERFAHREN UND VORRICHTUNG ZUR HERSTELLUNG EINES VERBUNDSTOFFS AUS DURCHLAUFENDEN FOLIEN FÜR EINEN SAUGFÄHIGEN ARTIKEL
PROCÉDÉ ET DISPOSITIF DE FABRICATION D'UN COMPOSITE À BASE DE FEUILLES CONTINUES POUR ARTICLE ABSORBANT

(30) Priority: 19.10.2009 JP 2009240709
(43) Date of publication of application: 29.08.2012
(73) Proprietor: Uni-Charm Corporation, Ehime 799-0111 (JP)
(72) Inventor: OGASAWARA, Yoshikazu, Kanonji-shi Kagawa 769-1602 (JP); MIYAZAKI, Kazuyo, Kanonji-shi Kagawa 769-1602 (JP)
(74) Representative: Eke, Philippa Dianne
(86) International application number: PCT/JP2010/067619
(87) International publication number: WO 2011/048954

(56) References cited:
- JP-A- 1 013 358
- JP-A- 7 309 432
- JP-A- 10 218 471
- JP-A- 10 218 471
- JP-A- 10 274 815
- JP-A- 11 347 504
- JP-A- 2002 035 027
- JP-A- 2002 104 320
- JP-A- 2005 199 395
- JP-A- 2005 335 041
- JP-A- 2005 521 612
- JP-A- 2006 062 839
- JP-A- 2007 260 413
- JP-A- 2007 260 875
- JP-A- 2007 260 875
- US-A1- 2003 047 273

## Description

### Technical Field ..

The present invention relates to a method and an apparatus for manufacturing a composite of a continuous sheet for an absorbent article such as a disposable diaper.

### Background Art

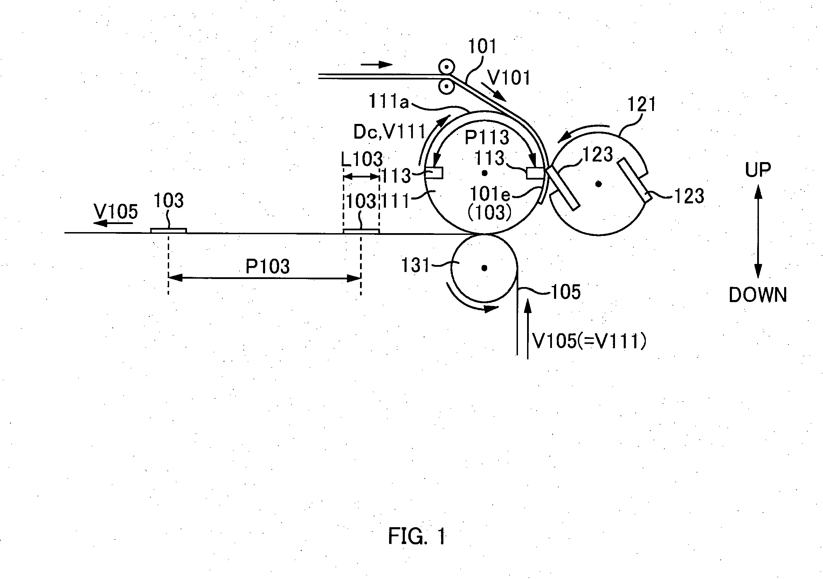

In a manufacturing line of an absorbent article such as a disposable diaper or a sanitary napkin, as shown in FIG. 1, a continuous film 101 is divided to produce single-cut films 103 having a predetermined length L103, and each of the produced single-cut films 103, 103...is adhered to a continuous sheet 105 such as a nonwoven fabric in a continuous direction thereof at a predetermined adhesion pitch P103.

As an example of this method, PTL 1 discloses a method using an anvil roll 111 that is driven to rotate in a circumferential direction Dc, a cutter roll 121 that is disposed facing the anvil roll 111 and rotates in synchronization with the anvil roll, and a transfer roll 131 disposed on a downstream side of the cutter roll 121 in the circumferential direction Dc.

To be specific, first, the continuous film 101 is supplied at a speed V101 to a peripheral surface 111a of the anvil roll 111 rotating at a predetermined peripheral speed V111, the speed being slower than the peripheral speed V111, and a part 101e on a leading end of the continuous film 101 is held on the peripheral surface 111a in a face contact state while sliding by a suction section of the peripheral surface acting thereto. Next, in a case where a cutter receiving part 113 on the peripheral surface 111a of the anvil roll 111 passes a position of the cutter roll 121, the continuous film 101 is divided and the part 101e on the leading end is cut and separated by a blade 123 of the cutter roll 121 and the cutter receiving part 113, and thereby the single-cut film 103 is produced. Then, the produced single-cut film 103 is transported in the circumferential direction Dc at the peripheral speed V111 of the anvil roll 111 while being held by the suction section of the peripheral surface 111a of the anvil roll 111. And when the single-cut film 103 passes a position that faces the transfer roll 131 in a transport path in the circumferential direction Dc, the single-cut film 103 is adhered to the continuous sheet 105 transported on the transfer roll 131.

In the method of PTL 1, a transport speed V105 of the continuous sheet 105 and the peripheral speed V111 of the anvil roll 111 are both set as the same speed.

### Citation List

### Patent Literature

PTL 1: JP-A-H10-218471

### SUMMARY OF INVENTION

### Technical Problem

On the other hand, the size of a product is generally changed in a production line. For example, in a case the size is changed from small to large, the length L103 of the single-cut film 103 and the adhesion pitch P103 are changed to become longer.

Here, change in the former length L103 of the single-cut film 103 can be easily met by increase-decrease adjustment of the supply speed V101 of the continuous film 101 with respect to the peripheral speed V111 of the anvil roll 111. For example, the supply speed V101 should be increased in a case of elongating the length L103 of the single-cut film 103, and in contrast, the supply speed V101 should be decreased in a case of shortening the length L103.

However, the latter adhesion pitch P103 cannot be easily changed as in the case mentioned above. That is, when changing the adhesion pitch P103 under a constraint that "the peripheral speed V111 of the anvil roll 111 and the transport speed V105 of the continuous sheet 105 are both the same" as in PTL 1, the anvil roll 111 needs to be changed to that having a roll diameter that corresponds to such adhesion pitch P103. This is because, the adhesion pitch P103 is uniquely determined by a disposition pitch P113 of the cutter receiving part 113 of the anvil roll 111 in the circumferential direction Dc since the peripheral speed V111 of the anvil roll 111 and the transport speed V105 of the continuous sheet 105 are both the same.

This results in the need for roll change equipment for changing the product sizes thus making the facilities complicated, and the facility operation rate will drop due to the regular roll exchange work required.

The present invention was made in view of the foregoing issue, and it is an advantage thereof to provide a method and an apparatus for manufacturing a composite of a continuous sheet for an absorbent article that can change the product size without any roll exchange.

### Solution to Problem

A main aspect of the invention for solving the foregoing issue is method of manufacturing a composite of a continuous sheet for an absorbent article, dividing and producing from a first continuous sheet single-cut sheets of a predetermined length, and adhering the single-cut sheets to a second continuous sheet in a continuous direction thereof at a predetermined adhesion pitch, including:
holding the first continuous sheet on a peripheral surface of a roll while sliding, by supplying the first continuous sheet continuously on the peripheral surface of the roll at a first speed lower than a peripheral speed of the roll;
producing the single-cut sheet by dividing the first continuous sheet with a cutter at a time a cutter receiving part provided on the peripheral surface passes a position of the cutter disposed to face the peripheral surface at a predetermined position in a peripheral direction of the roll;
transporting in the peripheral direction and at the peripheral speed the produced single-cut sheet held on the peripheral surface;
selecting one continuous sheet among a plurality of continuous sheets as the second continuous sheet, the plurality of continuous sheets including a continuous sheet transported at a second speed that is higher than the peripheral speed, and a continuous sheet transported at a third speed that is equal to or higher than the peripheral speed but lower than the second speed; and
adhering the single-cut sheet on the peripheral surface to the continuous sheet by supplying the selected continuous sheet towards the peripheral surface of the roll rotating at the peripheral speed, while coinciding a transport direction of the selected continuous sheet with a rotating direction of the roll.

And also a main aspect of the invention for solving the foregoing issue is
an apparatus for manufacturing a composite of a continuous sheet for an absorbent article, dividing and producing from a first continuous sheet single-cut sheets of a predetermined length, and adhering the single-cut sheets to a second continuous sheet in a continuous direction thereof at a predetermined adhesion pitch, comprising:
a roll that is driven to rotate about a predetermined rotational axis at a predetermined peripheral speed;
a first supply mechanism that supplies the first continuous sheet to a peripheral surface of the roll;
a cutter disposed to face the peripheral surface at a predetermined position in a peripheral direction of the roll;
a cutter receiving part that is provided on the peripheral surface of the roll and divides the first continuous sheet in cooperation with the cutter; and
a second supply mechanism that selects one continuous sheet among a plurality of continuous sheets as the second continuous sheet, the plurality of continuous sheets including a continuous sheet transported at a second speed that is higher than the peripheral speed, and a continuous sheet transported at a third speed that is equal to or higher than the peripheral speed but lower than the second speed, and supplies the selected continuous sheet to the peripheral surface;
wherein the first supply mechanism holds the first continuous sheet on the peripheral surface while sliding, by continuously supplying the first continuous sheet at a first speed lower than the peripheral speed of the roll,
the cutter produces the single-cut sheet by dividing the first continuous sheet in cooperation with the cutter receiving part at a time the cutter receiving part passes a position of the cutter,
the roll transports while holding on the peripheral surface thereof the produced single-cut sheet at the peripheral speed, and
the second supply mechanism supplies the selected continuous sheet towards the peripheral surface of the roll rotating at the peripheral speed and adheres the single-cut sheet on the peripheral surface to the continuous sheet while coinciding a transport direction of the selected continuous sheet with a rotating direction of the roll.

Other features of the invention will become clear by the description of the present specification and the accompanying drawings.

### Advantageous Effects of Invention

According to the present invention, the product size can be changed without any roll exchange when manufacturing a composite of a continuous sheet for an absorbent article.

### BRIEF DESCRIPTION OF DRAWINGS

FIG. 1 is an explanatory diagram of a conventional method and an apparatus for manufacturing.
FIG. 2A is a schematic planar view of a back face sheet 1 and an intermediate component 1a that is a basis of the back face sheet 1.
FIG. 2B is a schematic planar view of a back face sheet 1 and an intermediate component 1a that is a basis of the back face sheet 1.
FIG. 3 is a schematic side view of a manufacturing apparatus 10 used in the method of manufacturing according to the present embodiment.
FIG. 4 is a schematic side view of the manufacturing apparatus 10 in a case of manufacturing the intermediate component 1a of a small size.
FIG. 5 is a schematic side view of the manufacturing apparatus 10 in a case of manufacturing the intermediate component 1a of a large size.
FIG. 6 is an explanatory diagram of an example of areas in which an air intake operation is turned ON/OFF in a circumferential direction Dc of an anvil roll 11.
FIG. 7 is an explanatory diagram of a disposition pattern of air intake holes 13 on a peripheral surface 11a of the anvil roll 11, and a flattened view of the peripheral surface 11a in the circumferential direction Dc.
FIG. 8 is an enlarged side view of an end holding area A3e of the anvil roll 11.
FIG. 9 is an enlarged side view of a remaining area A3r of the anvil roll 11.
FIG. 10 is an explanatory diagram of a suction belt conveyor 43 having an endless belt 44 provided with a protruded part 44p.
FIG. 11 is an explanatory diagram of a hammer roll 51 as an example of another embodiment.

### DESCRIPTION OF EMBODIMENTS

At least the following matters will be made clear by the description in the present specification and the accompanying drawings.

A method of manufacturing a composite of a continuous sheet for an absorbent article, dividing and producing from a first continuous sheet single-cut sheets of a predetermined length, and adhering the single-cut sheets to a second continuous sheet in a continuous direction thereof at a predetermined adhesion pitch, including:
holding the first continuous sheet on a peripheral surface of a roll while sliding, by supplying the first continuous sheet continuously on the peripheral surface of the roll at a first speed lower than a peripheral speed of the roll;
producing the single-cut sheet by dividing the first continuous sheet with a cutter at a time a cutter receiving part provided on the peripheral surface passes a position of the cutter disposed to face the peripheral surface at a predetermined position in a peripheral direction of the roll;
transporting in the peripheral direction and at the peripheral speed the produced single-cut sheet held on the peripheral surface;
selecting one continuous sheet among a plurality of continuous sheets as the second continuous sheet, the plurality of continuous sheets including a continuous sheet transported at a second speed that is higher than the peripheral speed, and a continuous sheet transported at a third speed that is equal to or higher than the peripheral speed but lower than the second speed; and
adhering the single-cut sheet on the peripheral surface to the continuous sheet by supplying the selected continuous sheet towards the peripheral surface of the roll rotating at the peripheral speed, while coinciding a transport direction of the selected continuous sheet with a rotating direction of the roll.

According to such method of manufacturing a composite of a continuous sheet, a change of product size can be easily managed. That is, in a case of changing a length of the single-cut film along with changing the product size, the first speed of the first continuous sheet should be relatively-changed with respect to the peripheral speed of the roll. Also, the adhesion pitch of the single-cut film can be changed by at least selecting either of the continuous sheets transported at the second speed or the continuous sheet transported at the third speed as the second continuous sheet. Thus, the product size can be changed without any roll exchange.

In the method of manufacturing a composite of a continuous sheet for an absorbent article, it is preferable that
in a case the continuous sheet transported at the second speed is selected as the second continuous sheet in the selecting,
in the adhering,
a leading end in the peripheral direction of the single-cut sheet is adhered to the second continuous sheet, and thereafter, the single-cut sheet is pulled by the second continuous sheet via the leading end, and while a part of the single-cut sheet held on the peripheral surface slides relatively with respect to the peripheral surface in a travelling direction, the part is gradually peeled off from the peripheral surface to be overlapped and adhered onto the second continuous sheet.

According to this method of manufacturing a composite of a continuous sheet, the single-cut sheet moves together with the peripheral surface of the roll at the peripheral speed before adhesion of the leading end of the single-cut sheet to the second continuous sheet, however, after the adhesion of the leading end to the second continuous sheet, the single-cut sheet can move together with the second continuous sheet at the second speed as the transport speed of the second continuous sheet by sliding relatively with respect to the peripheral surface. In this way, unreasonable load caused by relative speed difference between the peripheral speed of the peripheral surface and the second speed of the second continuous sheet is prevented from acting on the single-cut sheet when being handed over between the two. And as a result, generation of wrinkles on the single-cut sheet when being adhered to the second continuous sheet is prevented.

In the method of manufacturing a composite of a continuous sheet for an absorbent article, it is preferable that
the single-cut sheet moves together with the peripheral surface at the peripheral speed until the leading end part of the single-cut sheet is adhered to the second continuous sheet, and the single-cut sheet moves together with the second continuous sheet at the second speed while sliding relatively with respect to the peripheral surface in the travelling direction after the leading end part is adhered to the second continuous sheet.

According to this method of manufacturing a composite of a continuous sheet, the single-cut sheet can be smoothly handed over from the peripheral surface to the second continuous sheet.

In the method of manufacturing a composite of a continuous sheet for an absorbent article, it is preferable that
the peripheral surface includes a leading end holding area that holds the leading end, and a rear part holding area that holds a part rear of the leading end in the peripheral direction, and
a holding force per unit area for holding the single-cut sheet on the peripheral surface is smaller in the rear part holding area than in the leading end holding area.

According to this method of manufacturing a composite of a continuous sheet, the rear part can relatively slide smoothly with respect to the peripheral surface that should be performed after adhesion of the leading end to the second continuous sheet.

Also, since the holding force in the end holding area is high, the leading end can be effectively prevented from being peeled that may be caused by air resistance or the like in a case where the single-cut sheet being held on the peripheral surface is transported at the peripheral speed. As a result, adhesion deficiency of the single-cut sheet to the second continuous sheet can be prevented.

In the method of manufacturing a composite of a continuous sheet for an absorbent article, it is preferable that
the peripheral surface includes the leading end holding area that holds the leading end part and a remaining area that is other than the leading end part holding area, and
the leading end holding area includes a protruded part protruding outward in a radial direction of the roll beyond the remaining area.

According to this method of manufacturing a composite of a continuous sheet, when the leading end of the single-cut sheet is adhered to the second continuous sheet, the leading end can be pressed against the second continuous sheet by the protruded part of the end holding area. As a result, adhesion strength between the leading end and the second continuous sheet can be increased.

Also, the remaining area is relatively distant from the second continuous sheet than the end holding area by an amount of at least the length of the protruded part. Thus, contact between the second continuous sheet and the peripheral surface is suppressed while increasing the pressing force applied to the leading end. And in this way, it is possible to control scratch damage on the surface of the second continuous sheet that may be caused by the relative speed difference between the second continuous sheet and the peripheral surface.

In the method of manufacturing a composite of a continuous sheet for an absorbent article, it is preferable that
the peripheral surface includes a width direction that is perpendicular to the peripheral direction,
a plurality of air intake holes are formed on the peripheral surface and the single-cut sheet is attracted and held on the peripheral surface by an air intake through the air intake holes, and
a groove part is formed on the peripheral surface to connect at least some of the air intake holes in a breathable manner, and a part of the groove part is positioned on an outer side of the single-cut sheet in the width direction.

According to this method of manufacturing a composite of a continuous sheet, the air intake hole connected to the groove part takes in outside air from the part of the groove part with relatively small resistance, and firm adsorption that may be caused by vacuum when the air intake hole is blocked by the single-cut sheet can be avoided. As a result, the peripheral surface is prevented from holding the single-cut sheet firmly, and in the aforementioned "adhering", a peeling-off resistance when peeling off the single-cut sheet from the peripheral surface is reduced, and thereby the single-cut sheet can be handed over from the peripheral surface to the second continuous sheet smoothly.

In the method of manufacturing a composite of a continuous sheet for an absorbent article, it is preferable that
the peripheral surface includes the leading end holding area that holds the leading end, and the rear part holding area that holds the part rear of the leading end in the peripheral direction, and
the groove part is connected to some of the air intake holes positioned in the rear part holding area in a breathable manner.

According to this method of manufacturing a composite of a continuous sheet, a firm adsorption alike vacuuming of the single-cut sheet that may occur in the rear holding area is prevented effectively. Thus, the rear part of the single-cut sheet can relatively slide with respect to the rear holding area smoothly which should be performed after adhesion of the leading end to the second continuous sheet.

In the method of manufacturing a composite of a continuous sheet for an absorbent article, it is preferable that
the peripheral surface includes a width direction that is perpendicular to the peripheral direction,
a plurality of air intake holes are formed on the peripheral surface and the single-cut sheet is attracted and held on the peripheral surface by the air intake through the air intake holes,
at an inside of the roll, at least some of the air intake holes are in communication with each other through a communication path in a breathable manner, and
some of the air intake holes in communication with each other through the communication path are positioned on an outer side of the single-cut sheet in the width direction.

According to this method of manufacturing a composite of a continuous sheet, of the air intake holes connected to the communication path, those positioned outside in the width direction takes in outside air through the air intake holes with relatively small resistance, and the firm adsorption that may be caused by vacuum by the air intake hole being blocked by the single-cut sheet can be avoided. As a result, the peripheral surface is prevented from holding the single-cut sheet firmly, and in the aforementioned "adhering", a peeling-off resistance when peeling off the single-cut sheet from the peripheral surface is reduced, and thereby the single-cut sheet can be handed over from the peripheral surface to the second continuous sheet smoothly.

In the method of manufacturing a composite of a continuous sheet for an absorbent article, it is preferable that
the peripheral surface includes the leading end holding area that holds the leading end, and the rear part holding area that holds the part rear of the leading end part in the peripheral direction, and
the communication path is in communication with some of the air intake holes positioned in the rear part holding area in a breathable manner.

According to this method of manufacturing a composite of a continuous sheet, the firm adsorption alike vacuuming of the single-cut sheet that may occur in the rear holding area is prevented effectively. Thus, the rear part of the single-cut sheet can relatively slide with respect to the rear holding area smoothly which should be performed after adhesion of the leading end to the second continuous sheet.

In the method of manufacturing a composite of a continuous sheet for an absorbent article, it is preferable that
the second continuous sheet has a higher air permeability than the single-cut sheet,
a transport path of the second continuous sheet is in a direction parallel to a tangent direction of the peripheral surface,
an air intake mechanism is provided to an adjacent position of the transport path closest to the peripheral surface to perform through the second continuous sheet an air intake in a direction that separates the single-cut sheet from the peripheral surface, and
in the adhering,
at a time each portions of the peripheral surface passes the adjacent position, a holding force of each of the portions that holds the single-cut sheet is reduced, and thereby a portion of the single-cut sheet that passes the adjacent position transfers gradually from the peripheral surface to the second continuous sheet.

According to this method of manufacturing a composite of a continuous sheet, the suction force by the air intake of the air intake mechanism at the adjacent position can act on the single-cut sheet through the second continuous sheet based on the high air permeability of the second continuous sheet. And the holding force of each of the portions of the peripheral surface is weakened when passing the adjacent position. Thus, the single-cut sheet can be transferred from the peripheral surface to the second continuous sheet smoothly at the adjacent position.

In the method of manufacturing a composite of a continuous sheet for an absorbent article, it is preferable that
the air intake mechanism is a suction belt conveyor that transports the second continuous sheet,
the suction belt conveyor includes a belt having a plurality of air intake holes, the belt moving along the transport path while attracting the second continuous sheet by an air intake through the air intake holes, and
in the adhering,
a suction force by the air intake through the air intake holes of the belt acts, through the second continuous sheet, on a portion of the single-cut sheet that is transferred from the peripheral surface to the second continuous sheet.

According to this method of manufacturing a composite of a continuous sheet, the single-cut sheet can be attracted to the belt through the second continuous sheet. Thus, the load that is needed for peeling off the single-cut sheet from the peripheral surface after adhering the leading end of the single-cut sheet to the second continuous sheet can be imposed on the belt 44. As a result, the load on the second continuous sheet is reduced, and generation of wrinkles on the second continuous sheet can be suppressed.

In the method of manufacturing a composite of a continuous sheet for an absorbent article, it is preferable that
the peripheral surface includes the leading end holding area that holds the leading end and the remaining area that is other than the leading end holding area, and
a space between the remaining area and the belt is larger than a sum of a thickness of the single-cut sheet and a thickness of the second continuous sheet.

According to this method of manufacturing a composite of a continuous sheet, the contact between the remaining area and the second continuous sheet can be reduced, and it is possible to inhibit scratch damage on the surface of the second continuous sheet that may be caused by the relative speed difference between the second continuous sheet and the remaining area.

In the method of manufacturing a composite of a continuous sheet for an absorbent article, it is preferable that
the peripheral surface of the roll includes the leading end holding area that holds the leading end, and a remaining area that is other than the leading end holding area,
the second continuous sheet has a higher air permeability than the single-cut sheet,
a transport path of the second continuous sheet is in a direction parallel to a tangent direction of the peripheral surface,
in an adjacent position closest the peripheral surface of the transport path, an air intake mechanism is provided to perform an air intake in a direction that separates the single-cut sheet from the peripheral surface through the second continuous sheet,
the air intake mechanism is a suction belt conveyor that transports the second continuous sheet,
the suction belt conveyor includes a belt having a plurality of air intake holes, the belt moving along the transport path while attracting the second continuous sheet to a peripheral surface thereof by the air intake through the air intake holes, and
a protruded part is provided at a portion in the peripheral surface of the belt that should face the leading end holding area of the roll.

According to this method of manufacturing a composite of a continuous sheet, in the case of adhering the leading end of the single-cut sheet to the second continuous sheet, the leading end is attracted toward the second continuous sheet by the air intake of the belt of the suction belt conveyor, and in addition to this, the second continuous sheet can be pressed against the leading end by the protrusion of the belt. As a result, adhesion strength between the leading end and the second continuous sheet can be increased.

In the method of manufacturing a composite of a continuous sheet for an absorbent article, it is preferable that
the third speed is same as the peripheral speed,
in the selecting, in a case where the continuous sheet transported at the third speed is selected as the second continuous sheet, the second continuous sheet is transported toward the roll along a transport path that wraps around the peripheral surface at a predetermined wrapping angle, and
in the adhering, the single-cut sheet adheres to the second continuous sheet while wrapping around at the wrapping angle.

According to this method of manufacturing a composite of a continuous sheet, in the case where the continuous sheet transported at the third speed is selected as the second continuous sheet, the transport speed of the second continuous sheet becomes the same speed as the peripheral speed of the peripheral surface, and the relative speed difference between the single-cut sheet held on the peripheral surface and the second continuous sheet is nearly nil. Thus, generation of wrinkles when adhering the single-cut sheet to the second continuous sheet is suppressed efficiently.

Also, an apparatus for manufacturing a composite of a continuous sheet for an absorbent article, dividing and producing from a first continuous sheet single-cut sheets of a predetermined length, and adhering the single-cut sheets to a second continuous sheet in a continuous direction thereof at a predetermined adhesion pitch, including:
a roll that is driven to rotate about a predetermined rotational axis at a predetermined peripheral speed;
a first supply mechanism that supplies the first continuous sheet to a peripheral surface of the roll;
a cutter disposed to face the peripheral surface at a predetermined position in a peripheral direction of the roll;
a cutter receiving part that is provided on the peripheral surface of the roll and divides the first continuous sheet in cooperation with the cutter; and
a second supply mechanism that selects one continuous sheet among a plurality of continuous sheets as the second continuous sheet, the plurality of continuous sheets including a continuous sheet transported at a second speed that is higher than the peripheral speed, and a continuous sheet transported at a third speed that is equal to or higher than the peripheral speed but lower than the second speed, and supplies the selected continuous sheet to the peripheral surface;
wherein the first supply mechanism holds the first continuous sheet on the peripheral surface while sliding, by continuously supplying the first continuous sheet at a first speed lower than the peripheral speed of the roll,
the cutter produces the single-cut sheet by dividing the first continuous sheet in cooperation with the cutter receiving part at a time the cutter receiving part passes a position of the cutter,
the roll transports while holding on the peripheral surface thereof the produced single-cut sheet at the peripheral speed, and
the second supply mechanism supplies the selected continuous sheet towards the peripheral surface of the roll rotating at the peripheral speed and adheres the single-cut sheet on the peripheral surface to the continuous sheet while coinciding a transport direction of the selected continuous sheet with a rotating direction of the roll.

According to such an apparatus for manufacturing a composite of a continuous sheet, operational advantages that are the same as the above-mentioned method of manufacturing can be realized.

### === Present Embodiment ===

In a method of manufacturing a composite of a continuous sheet for an absorbent article according to a first embodiment, an intermediate component 1a that becomes a basis of a back face sheet 1 of a disposable diaper is manufactured as an example of the composite of the continuous sheet.

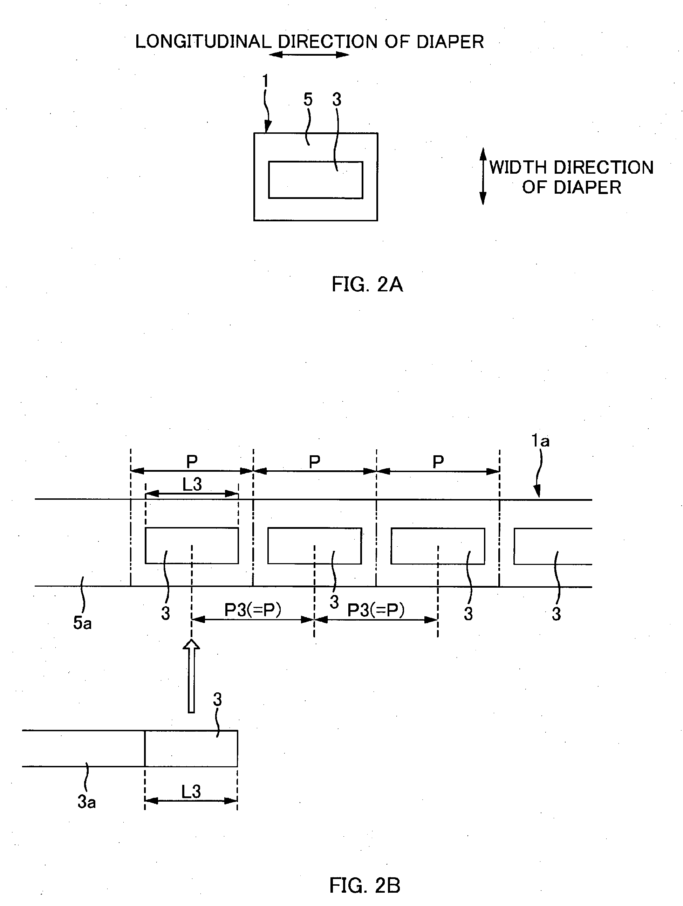

FIGS. 2A and 2B respectively show schematic planar views of the back face sheet 1 and the intermediate component 1a that becomes the basis of the back face sheet 1.

The back face sheet 1 shown in FIG. 2A is a composite sheet 1 that includes an exterior sheet 5 forming an exterior of the diaper, an impermeable leak-proof film 3 that is adhered to a face in an inner side of the exterior sheet 5 (face on a side of a wearer's skin). On top of the leak-proof film 3, an absorbent body that is not shown formed by molding a pulp fiber, a permeable surface sheet that is also not shown and the like are successively stacked and fixed, and become a basis of the diaper.

A nonwoven fabric that includes a resin fiber as main material or the like can be given as an example of a material of the exterior sheet 5, and here, it is the nonwoven fabric. Also, a resin film or the like can be given as an example of a material of the leak-proof film 3, and here, it is the resin film.

From the viewpoint of cost reduction, the planar size of the leak-proof film 3 is smaller than the planar size of the exterior sheet 5. Also, air permeability of the leak-proof film 3 in the thickness direction (direction that penetrates the paper surface) is lower than air permeability of the exterior sheet 5 in the thickness direction.

As shown in FIG. 2B, the intermediate component 1a that becomes the basis of the back face sheet 1 is a continuous body before being divided into back face sheets 1 at a product pitch P. That is, the intermediate component 1a is made by intermittently adhering a plurality of leak-proof films 3, 3... on the continuous sheet 5a of the nonwoven fabric as original cloth of the exterior sheet 5 in a continuing direction thereof at an adhesion pitch P3 having a same value as the product pitch P.

Thus, the method of manufacturing the intermediate component 1a includes a process of dividing a continuous film 3a (corresponds to a first continuous sheet) as an original cloth of the leak-proof film 3 and thereby producing a single-cut film 3 (corresponds to a single-cut sheet) having a predetermined length of L3, and a process of adhering the produced single-cut film 3 as the leak-proof film 3 to the continuous sheet 5a (corresponds to a second continuous sheet) as an original cloth of the exterior sheet 5 in the continuing direction thereof at the above mentioned adhesion pitch of P3.

In such method of manufacturing, the length L3 of the single-cut film 3 and the adhesion pitch P3 need to be changed for changing the product size. However, by using the method of manufacturing according to the present embodiment, such requirements can be easily met without any exchanging of large-scale equipment and the like such as roll exchange and the like as explained below.

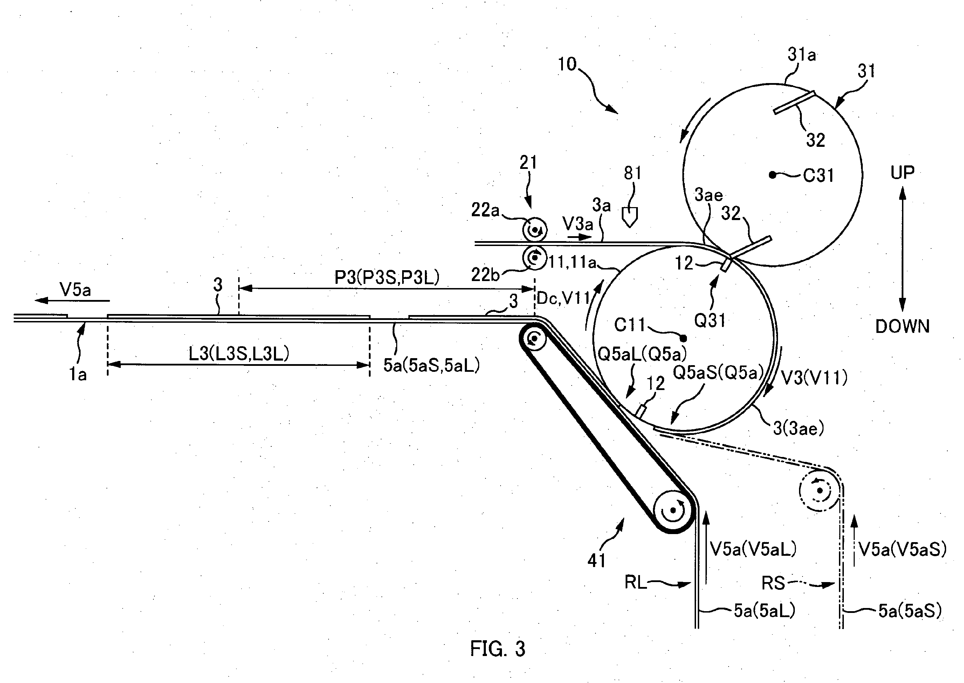

FIG. 3 is a schematic side view of an apparatus 10 for manufacturing used in the method of manufacturing according to the present embodiment. Hereafter, a width direction of the continuous sheet 5a is referred to as the CD direction, and this CD direction is perpendicular to a transport direction (continuous direction) of the continuous sheet 5a, and points a direction that penetrates the paper surface in FIG. 3.

The apparatus 10 for manufacturing includes, (1) an anvil roll 11 that is driven to rotate about a rotational axis C11 pointing the CD direction at a predetermined peripheral speed V11 in a circumferential direction Dc, (2) a continuous film supply mechanism 21 that continuously supplies the continuous film 3a to a peripheral surface 11a of the anvil roll 11 at a supply speed V3a slower than the peripheral speed V11, (3) a cutter roll 31 that is disposed to face the anvil roll 11 at a predetermined position Q31 in the circumferential direction Dc and divides the continuous film 3a in cooperation with the anvil roll 11 and thereby produces the single-cut film 3, and (4) a continuous sheet transport mechanism 41 that continuously supplies the continuous sheet 5a toward the peripheral surface 11a of the anvil roll 11 while coinciding a transport direction thereof with a rotating direction of the anvil roll 11 for the purpose of adhering the single-cut film 3 held on the peripheral surface 11a of the anvil roll 11 to the continuous sheet 5a.

Here, the aforementioned affixation of the single-cut film 3 on the continuous sheet 5a is performed by adhesion. That is, before adhesion, an adhesive is pre-applied on at least either of the faces to be adhered to each other, which is the continuous sheet 5a or the single-cut film 3. In this example, as shown in FIG. 3, a hot-melt adhesive is applied on a substantially entire surface of one side of the continuous film 3a by an adhesive applying system 81 right before supplying the continuous film 3a to the anvil roll 11.

Hereafter, explanation is given on each component 11, 21, 31, and 41.

The anvil roll 11 (corresponds to roll) is a cylindrical body having a perfect-circular cross section. At the peripheral surface 11a thereof, a receiving part 12 is provided for receiving a flat blade 32 of the cutter roll 31 (corresponds to a cutter receiving part). The receiving parts 12 are disposed at an equal pitch P12 in the circumferential direction Dc, and in the illustrated example, are disposed at two locations in the circumferential direction Dc. In this way, a sheet of single-cut film 3 is divided and produced by a half-turn of the anvil roll 11.

Also, the peripheral surface 11a has a function of holding sheet-type material by wrapping it around thereto in a state of face contact, and in this way, the single-cut film 3 that is divided and produced by the cutter roll 31, and a leading end 3ae of the continuous film 3a before being divided into the single-cut film 3 is held on the peripheral surface 11a in a state of face contact. In this example, this holding function is achieved by a plurality of air intake holes 13 formed on the peripheral surface 11a (not shown in FIG. 3). That is, suction force acts on the peripheral surface 11a of the anvil roll 11 by an air intake through the air intake holes 13, and this suction force becomes the holding force for holding the single-cut film 3 or the leading end 3ae of the continuous film 3a described above.

The continuous film supply mechanism 21 (corresponds to a first supply mechanism) has a pair of upper and lower pinch rolls 22a, 22b for example. And the pinch rolls 22a and 22b are driven to rotate while sandwiching the continuous film 3a therebetween, and supply the continuous film 3a to the peripheral surface 11a of the anvil roll 11 at the predetermined supply speed V3a.

Here, this supply speed V3a (corresponds to first speed) is set slower than the peripheral speed V11 of the anvil roll 11. Therefore, the leading end 3ae of the continuous film 3a is held on the peripheral surface 11a in a state of face contact while sliding in a direction to fall behind along the peripheral surface 11a of the anvil roll 11, until it is divided and separated from the continuous film 3a by the cutter roll 31. That is, the leading end 3ae of the continuous film 3a gradually moves toward a downstream side in the circumferential direction Dc while sliding on the peripheral surface 11a at the supply speed V3a. And as shown in FIG. 3, after being cut and separated from the continuous film 3a by the cutter roll 31, the leading end 3ae moves as the single-cut film 3 at the speed V3 same as the peripheral speed V11 of the anvil roll 11 while being held together on the peripheral surface 11a of the anvil roll 11. In this way, a space is generated between the succeeding single-cut film 3 that is divided and produced subsequently. And when reaching a supply position Q5a of the continuous sheet 5a set downstream in the circumferential direction Dc, the single-cut film 3 is adhered to the continuous sheet 5a and moves integral with the continuous sheet 5a.

The cutter roll 31 (corresponds to cutter) includes the roll 31 that is driven to rotate about a rotational axis C31 pointing the CD direction as a main body, and the flat blade 32 is provided on a peripheral surface 31a thereof. The cutter roll 31 is driven to rotate in synchronization with the anvil roll 11, and divides the leading end 3ae from the continuous film 3a in cooperation with the anvil roll 11 and thereby produces the single-cut film 3.

In detail, the cutter roll 31 is driven to rotate so that the flat blade 32 of the cutter roll 31 faces the receiving part 12 of the anvil roll 11 every time the receiving part 12 of the anvil roll 11 that rotates in the circumferential direction Dc passes the position Q31 of the cutter roll 31, and in this way the cutter roll 31 cuts and separates the leading end 3ae from the continuous film 3a in cooperation with the anvil roll 11. In the illustrated example, for the purpose of enabling such movement, a perimeter of the pitch circle of the flat blade 32 of the cutter roll 31 (path of an edge of the flat blade 32) and a perimeter of the pitch circle of the receiving part 12 of the anvil roll 11 (path of an edge of the receiving part 12) are set as a same value. And also, numbers of the flat blade 32 and the receiving part 12 are set the same being two.

The continuous sheet transport mechanism 41 (corresponds to second supply mechanism) includes, for example, a transport route RS for small size products for transporting a continuous sheet 5aS (5a) for small size products shown in a chain double-dashed line in FIG. 3, and a transport route RL for large size products for transporting a continuous sheet 5aL (5a) for large size products shown in a solid line in the same FIG. 3. Here, the transport route RL for large size products can supply the continuous sheet 5aL toward the peripheral surface 11a of the anvil roll 11 by setting a predetermined position in the circumferential direction Dc as a supply position Q5aL (Q5a) for large size products. On the other hand, the transport route RS for small size products can supply the continuous sheet 5aS toward the peripheral surface 11a by setting a position in the upstream side of the supply position Q5aL for large size products in the circumferential direction Dc as a supply position Q5aS (Q5a) for small size products. The transport route RS for small size products and the transport route RL for large size products are selected and used alternatively according to the change in product size. Due to the cooperation of this alternative selection and change in the supply speed V3a of the continuous film 3a made by the continuous film supply mechanism 21, the product size changed is performed as described below.

For example, in a case of manufacturing the intermediate component 1a for small size products, the continuous film supply mechanism 21 sets the supply speed V3a of the continuous film 3a to a slow speed V3aS for small size products. In this way, supply amount of the continuous film 3a per half-turn of the anvil roll 11 decreases, and the leading end 3ae of the continuous film 3a is divided into a short length L3S for small size products by the cutter roll 31 or the like, and as a result, the short single-cut film 3 for small size products is produced and held on the peripheral surface 11a of the anvil roll 11.

Meanwhile, the transport route RS for small size products is selected at the continuous sheet transport mechanism 41. In the transport route RS for small size products, the continuous sheet 5aS having narrow width that corresponds to small size products (corresponds to continuous sheet that is transported at a third speed) that is transported. Also, the continuous sheet 5aS is transported at a transport speed V5aS that is adapted to transport small size products (corresponds to the third speed) and in the example, it is transported at a same speed as the peripheral speed V11 of the anvil roll 11. In this way, on the continuous sheet 5aS, the short single-cut film 3 for small size products is adhered intermittently at an adhesion pitch P3S that is adapted to small size products and thereby the intermediate component 1a for small size products is manufactured.

On the other hand, in a case of manufacturing the intermediate component 1a for large size products, first, the continuous film supply mechanism 21 sets the supply speed V3a of the continuous film 3a to a speed V3aL faster than the supply speed V3aS for small size products. Thereby, supply amount of the continuous film 3a per half-turn of the anvil roll 11 increases, and the leading end 3ae of the continuous film 3a is divided into a long length L3L for large size products by the cutter roll 31 or the like, and as a result, the long single-cut film 3 for large size products is produced and held on the peripheral surface 11a of the anvil roll 11.

Meanwhile, the transport route RL for large size products is selected at the continuous sheet transport mechanism 41. In the transport route RL for large size products, the continuous sheet 5aL having broad width that corresponds to large size products (corresponds to continuous sheet that is transported in a second speed) is transported. Also, the continuous sheet 5aL is transported at a transport speed V5aL (corresponds to the second speed) that is adapted to transport large size products and is faster than the transport speed V5aS that is adapted to transport small size products. That is, in the example of FIG. 3, the continuous sheet 5aL is transported at the speed V5aL that is faster than the peripheral speed V11 of the anvil roll 11. In this way, on the continuous sheet 5aL, long single-cut films 3 for large size products are adhered intermittently at a long adhesion pitch P3L that is adapted to large size products and thereby the intermediate component 1a for large size is manufactured.

That is, the adhesion pitch P3L of large size products is further expanded than the adhesion pitch P3S of small size products based on a speed ratio R between the transport speed V5aL of large size products and the transport speed V5aS of small size products (=V5aL/V5aS) . In this way, together with the change in the length L3 of the single-cut film 3 (from L3S to L3L), change in the product size from small to large is achieved.

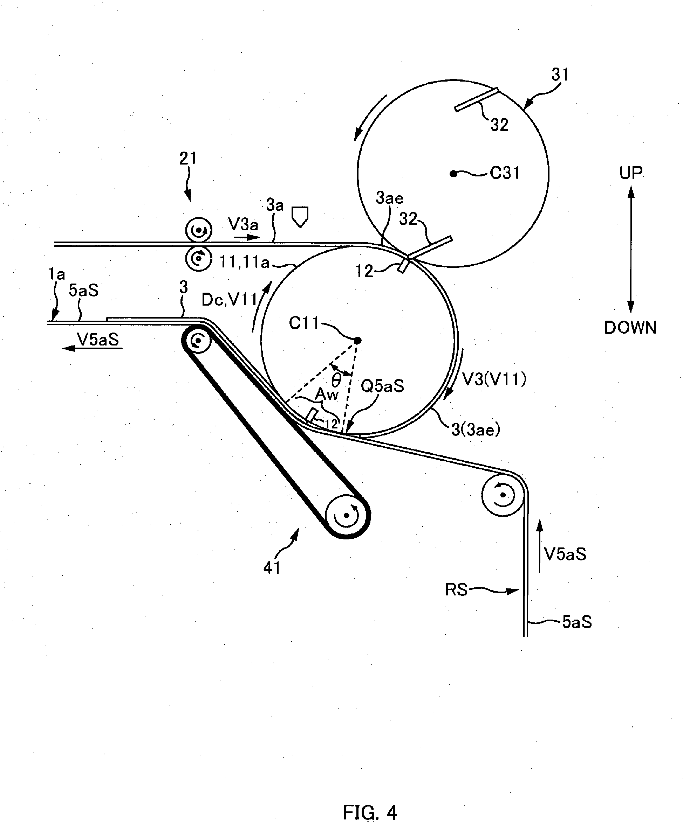

By the way, in the example, as shown in FIG. 4, the transport route RS for small size products is set as a transport path in which the continuous sheet 5aS for small size products is wrapped around the peripheral surface 11a of the anvil roll 11 at a predetermined wrapping angle θ. And in a case where the single-cut film 3 held on the peripheral surface 11a of the anvil roll 11 passes this wrapping-around range Aw, the single-cut film 3 is transferred from the peripheral surface 11a to the continuous sheet 5aS and is adhered to the continuous sheet 5aS, however, at the time of this transfer, the peripheral speed V11 of the anvil roll 11 and the transport speed V5aS of the continuous sheet 5aS are set to the same speed as described before. Therefore, there is no fear of the single-cut film 3 or the continuous sheet 5aS getting wrinkled caused by a relative speed difference during this transfer.

On the contrary, in the case for large size products, as shown in a solid line in FIG. 3, the peripheral speed V11 of the anvil roll 11 and the transport speed V5aL of the continuous sheet 5aL differ from each other. Here, in a case where the relative speed difference is small, wrinkles may not be obvious due to elastic deformation of the continuous sheet 5aL or the single-cut film 3. However, in a case where the relative speed difference is large, there is a high possibility of the single-cut film 3 or the continuous sheet 5aL getting wrinkled because of the speed difference during the transfer of the single-cut film 3.

To prevent the generation of wrinkles, in the present embodiment, several ingenuities are exercised in the continuous sheet transport mechanism 41 and in the anvil roll 11 as described below. Hereafter, these ingenuities will be explained.

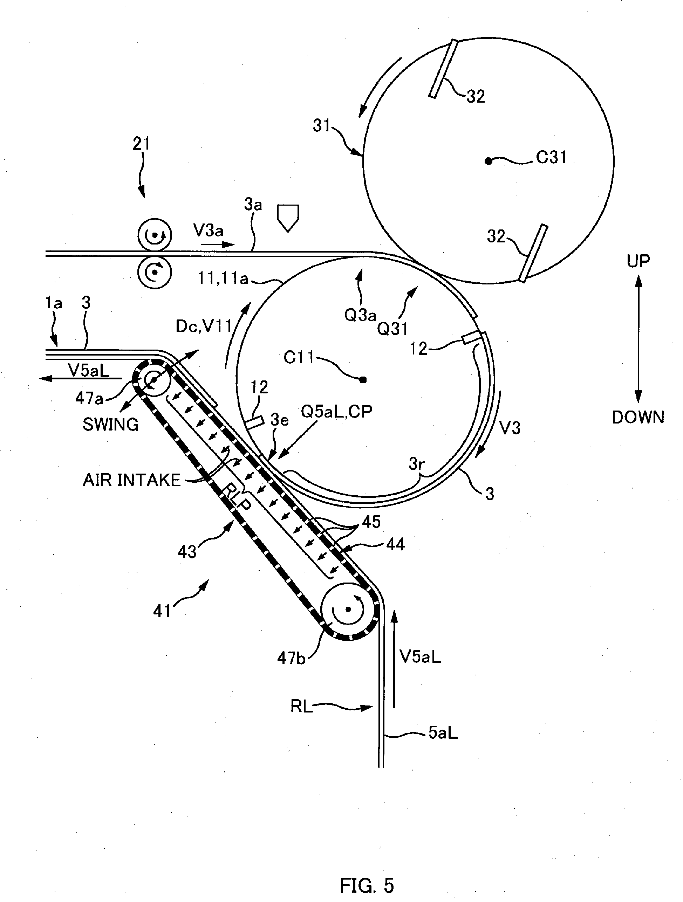

First, referring to FIG. 5, the ingenuities exercised on the continuous sheet transport mechanism 41 is explained. In the transport route RL for large size products formed by the continuous sheet transport mechanism 41, that is, in the transport path RL of the continuous sheet 5aL of large size products, a part RLP of the transport path is set along a direction parallel to a tangent direction of the peripheral surface 11a of the anvil roll 11. The transport path RLP is set as a route that most approaches the peripheral surface 11a at the supply position Q5aL in the circumferential direction Dc. Hereafter, the supply position Q5aL at which the closest approach is made in each of the transport route RL for large size products and the circumferential direction Dc is also referred to as "adjacent position CP".

In this way, in the case where the single-cut film 3 passes the adjacent position CP, first, the leading end 3e that is a downstream end in the circumferential direction Dc of the single-cut film 3 is adhered to the continuous sheet 5aL. After adhering the leading end 3e, since the transport speed V5aL of the continuous sheet 5aL is faster than the peripheral speed V11 of the anvil roll 11, the single-cut film 3 is pulled by the continuous sheet 5aL via the leading end 3e, and thereby a part 3r of the single-cut film 3 that is held on the peripheral surface 11a slides relatively with respect to the peripheral surface 11a in a travelling direction. And while sliding, the part 3r is gradually peeled off from the peripheral surface 11a and is overlapped and adhered on the continuous sheet 5aL.

That is, as shown in FIG. 3, before the adhesion of the leading end part 3e, the single-cut film 3 moves together with the peripheral surface 11a at the peripheral speed V11 of the peripheral surface 11a, however, after the adhesion of the leading end 3e as shown in FIG. 5, the single-cut film 3 moves together with the continuous sheet 5aL at the transport speed V5aL of the continuous sheet 5aL by sliding relatively with respect to the peripheral surface 11a in the travelling direction. In this way, unreasonable pulling-load caused by relative speed difference between the continuous sheet 5aL and the peripheral face 11a acting on the single-cut film 3 is suppressed. And as a result, generation of wrinkles at the time of adhesion is prevented. Further, ingenuities exercised on the anvil roll 11 described later also contribute largely to the relative sliding, and this will be described later.

In the example of FIG. 5, a suction belt conveyor 43 (corresponds to air intake mechanism) is used as a transport mechanism of the transport route RL for large size products, for the purpose of performing such hand over of the single-cut film 3 via the leading end part 3e.

More specifically, the conveyor 43 includes an endless belt 44 that travels in a predetermined orbit, and a plurality of air intake holes 45, 45... are formed on approximately the entire surface of a mounting face of the belt 44. And by the air intake through the air intake holes 45, 45... the continuous sheet 5aL is attracted to the mounting face. Here, a part of the orbit is set along the direction parallel to the tangent direction of the peripheral surface 11a of the anvil roll 11. Thereby, as described before, the part RLP of the transport path is set along the direction parallel to the tangent direction of the peripheral surface 11a of the anvil roll 11. Also, the air intake through each of the air intake holes 45, 45... continues while each portions of the belt 44 passes the adjacent position CP in the transport path RLP.

Thus, even in the case where the leading end 3e of the single-cut film 3 passes the adjacent position CP in the circumferential direction Dc, the air intake is performed through the continuous sheet 5aL having high air permeability in a direction that separates the single-cut film 3 from the peripheral surface 11a. And in this way, first, the leading end 3e of the single-cut film 3 is drawn toward the continuous sheet 5aL and adhered to the continuous sheet 5aL. And thereafter, each of the portions 3r on the rear side of the leading end 3e passes the adjacent position CP, however, also at that time, each of the portions 3r is drawn toward the continuous sheet 5aL successively by the air intake performed through the belt 44 and the continuous sheet 5aL having high air permeability, and is adhered to the continuous sheet 5aL.

Also, since the continuous sheet 5aL has a higher air permeability than the single-cut film 3, suction force caused by the air intake through the belt 44 wholly acts on the single-cut film 3 through the continuous sheet 5aL, to the portion of the single-cut film 3 that is transferred from the peripheral surface 11a to the continuous sheet 5aL. In this way, the single-cut film 3 is attracted to the belt 44 and thereby, a component of force needed for peeling off the single-cut film 3 from the peripheral surface 11a in the transport direction can be imposed on the belt 44. As a result, the load needed for the peeling-off that may be imposed on the continuous sheet 5aL mainly via the leading end 3e after adhering the leading end 3e can be imposed on the belt 44. And thus, the load on the continuous sheet 5aL is reduced and generation of wrinkles on the continuous sheet 5aL can be suppressed.

By the way, in the conveyor 43 of FIG. 5, the endless belt 44 can be configured so as to swing in a direction to separate from the anvil roll 11 by using either of the pair of pulleys 47a, 47b that form the orbit of the endless belt 44 as a fulcrum. For example, the pulley 47a positioned on a downstream side can be configured so as to swing by using the pulley 47b positioned on an upstream side of the adjacent position CP in the transport route RL for large size products as the fulcrum. By configuring so that, in a case of the leading end 3e of the single-cut film 3 passing the adjacent position CP, the endless belt 44 approaches the anvil roll 11 to press the continuous sheet 5aL to the leading end 3e, and on the other hand, after the leading end 3e has passed the adjacent position CP, the endless belt 44 is moved so as to separate from the anvil roll 11 to a passing position that is spaced by a predetermined distance, the leading end part 3e can be firmly adhered to the continuous sheet 5aL.

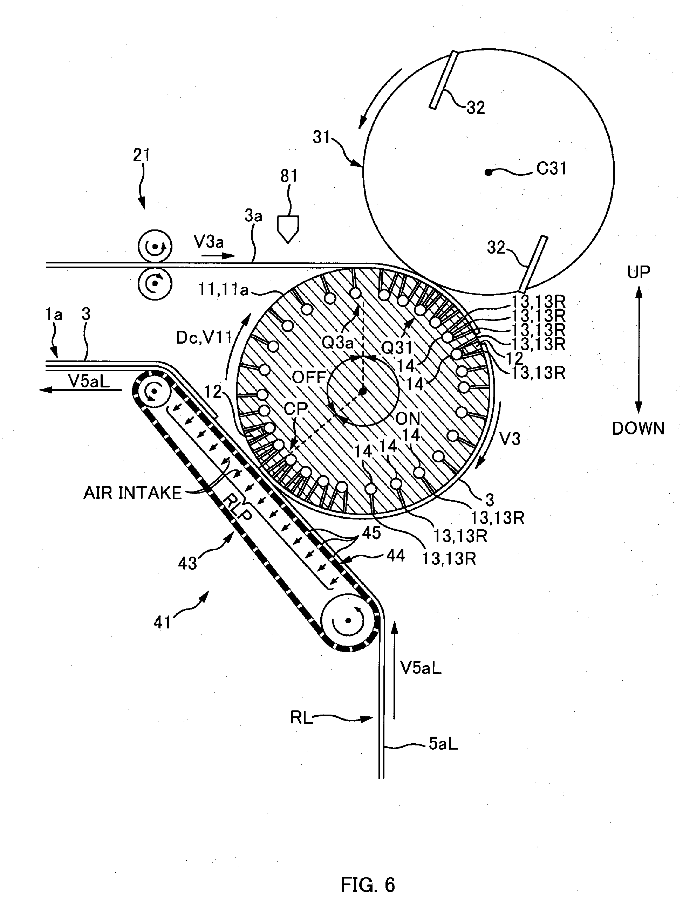

Next, ingenuities exercised on the anvil roll 11 are described. It has been mentioned above that the anvil roll 11 includes the plurality of air intake holes 13, 13... on the smooth peripheral surface 11a thereof, and the single-cut film 3 is attracted to the peripheral surface 11a by the air intake through these air intake holes 13, 13.... However, it is a matter of course that these air intake holes 13, 13... move in the circumferential direction Dc together with the peripheral surface 11a by rotation of the anvil roll 11. And during this moving process, each of the air intake holes 13, 13... is configured to turn on/off the air intake movement according to each position along the circumferential direction Dc. FIG. 6 is an explanatory diagram of an example of areas in which the air intake movement is turned on/off in the circumferential direction Dc. In the figure, inner configurations of the anvil roll 11 and the suction belt conveyor 43 are described.

As shown in FIG. 6, during the moving process of the air intake holes 13 in the circumferential direction Dc, the air intake through the air intake holes 13 is turned on in an area from a position Q3a where the continuous film 3a is supplied to the peripheral surface 11a, through the position Q31 of the cutter roll 31, to the adjacent position CP that corresponds to a transfer position of the single-cut film 3. However, when passing the adjacent position CP, the air intake of the air intake holes 13 is turned off. Therefore, when each portions of the peripheral surface 11a passes the adjacent position CP in the circumferential direction Dc, the air intake of the air intake hole 13 of each of the portions stops. Thereby, the part of the single-cut film 3 that has passed the adjacent position CP can smoothly transfer from the peripheral surface 11a to the continuous sheet 5aL successively. And when the air intake holes 13 returns to the supply position Q3a of the continuous film 3a by further moving in the circumferential direction Dc, the air intake through the air intake holes 13 turns on again and repeats the above mentioned movement.

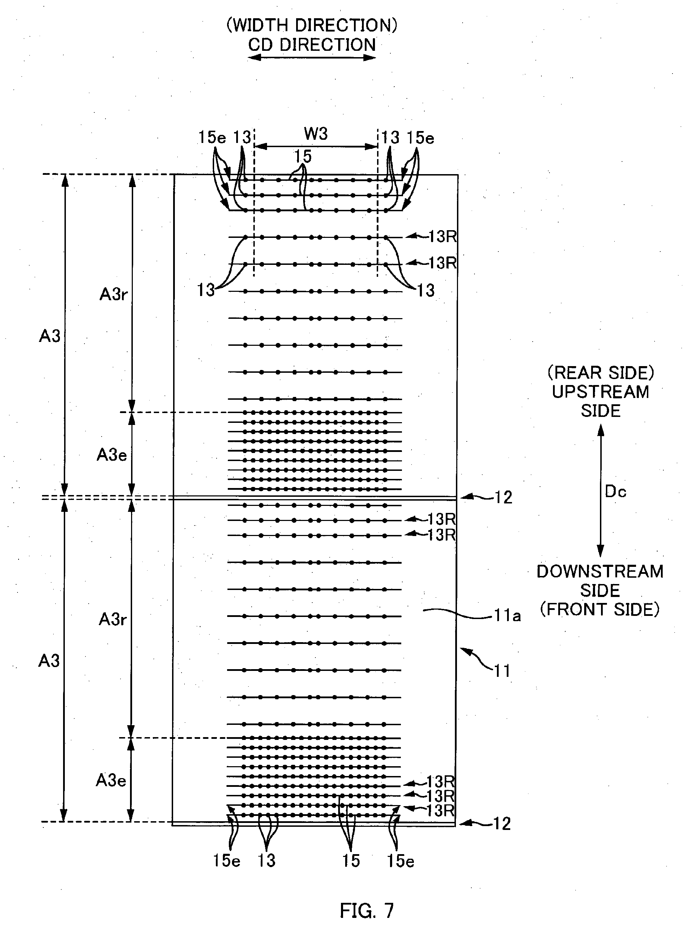

FIG. 7 is an explanatory diagram of a disposition pattern of the air intake holes 13 on the peripheral surface 11a of the anvil roll 11, and it is a developed view of the peripheral surface 11a in the circumferential direction Dc. In the example, the peripheral surface 11a has two areas A3, A3 aligned in the circumferential direction Dc that attract and hold the single-cut film 3. Each of the areas A3, A3 can hold one sheet of single-cut film 3. And each of the areas A3, A3 has a leading end holding area A3e and a rear part holding area A3r. The leading end holding area A3e is an area for attracting and holding the leading end 3e of the single-cut film 3, and is set for a predetermined area from a position nearest to the receiving part 12 on an upstream side as the start point to a further upstream side in the circumferential direction Dc. Whereas, the rear part holding area A3r is set further upstream of the leading end holding area A3e in the circumferential direction Dc and attracts and holds the part 3r that is at the rear of the leading end 3e of the single-cut film 3.

Here, as shown in FIG. 7, a disposition density of the air intake holes 13 (number of the air intake holes 13 disposed per unit area of the peripheral surface 11a) is lower in the rear part holding area A3r than in the leading end holding area A3e. Thus, a force for holding the single-cut film 3 per unit area is smaller in the rear part holding area A3r than in the leading end holding area A3e.

Thus, the rear part 3r can relatively slide with respect to the peripheral surface 11a smoothly which should be performed after adhesion of the leading end 3e to the continuous sheet 5aL. Also, in a case where the single-cut film 3 held on the peripheral surface 11a is integrally transported by the anvil roll 11 at the peripheral speed V11, there is fear of the leading end 3e of the single-cut film 3 being lifted by air resistance or the like. However, as described before, since the holding force in the leading end holding area A3e is increased, the leading end 3e is effectively prevented from being lifted. As a result, adhesion deficiency of the single-cut film 3 to the continuous sheet 5aL is prevented.

Also, if the air intake hole 13 is in a sealed state being blocked completely, vacuum is generated attracting the single-cut film 3 firmly. And the single-cut film 3 becomes difficult to peel off at the time of peeling off the single-cut film 3 from the peripheral surface 11a. And as a result, smooth transfer to the continuous sheet 5aL is impaired. Here, in the example of FIG. 7, a groove part 15 is formed on the peripheral surface 11a that connects at least some of the air intake holes 13, 13... in a breathable manner, and a part 15e of the groove part 15 is positioned outside the single-cut film 3 in the width direction (CD direction). Thereby outside air can be taken from the part 15e to the intake holes 13.

More specifically, in the example of FIG. 7, a plurality of rows 13R are aligned and disposed in the circumferential direction Dc, and the row 13R consists of the plurality of air intake holes 13, 13 aligned in the CD direction. Each of the rows 13R of air intake holes includes a groove part 15 along the CD direction, and thereby the air intake holes 13, 13 that belong to the same row 13R of air intake holes are in communication with each other through the groove part 15 in a breathable manner. In FIG. 7, a width size W3 of the single-cut film 3 is shown, and positions of both end parts 15e, 15e of the groove part 15 are located outside the single-cut film 3 in the width direction respectively. Thus, the air intake holes 13, 13... connected to the groove part 15 take in outside air through the both end parts 15e, 15e of the groove part 15 with relatively small resistance, and the air intake hole 13 is avoided from being in a vacuum sealed state even if the air intake holes 13 are completely covered with the single-cut film 3. That is, the peripheral surface 11a is prevented from holding the single-cut film 3 firmly and as a result, the peripheral surface 11a can hand over the single-cut film 3 to the continuous sheet 5aL smoothly.

By the way, such formation of the groove part 15 is effective especially to the row 13R of air intake holes positioned in the rear part holding area A3r. That is, it is preferable that the groove part 15 is connected to some of the air intake holes 13 positioned in the rear part holding area A3r in the breathable manner. In this way, the single-cut film 3 slides relatively with respect to the rear part holding area A3r smoothly that should be performed after adhering the leading end 3e of the single-cut film 3 to the continuous sheet 5aL. And as a result, generation of wrinkles on the single-cut film 3 can be suppressed more effectively.

By the way, in this example, since the disposition pattern of the air intake holes 13 of FIG. 7 is an approximately grid-style arrangement in which the air intake holes 13 are aligned in the circumferential direction Dc and in the CD direction, the groove part 15 is formed along the CD direction as in FIG. 7. However, the carving pattern of the groove part 15 is not limited to this. For example, in a case where the disposition pattern of the air intake holes 13 is in a so-called staggered arrangement, each of the air intake holes 13, 13... aligned in a zigzag-form in the CD direction can be connected by a groove part 15 in a zigzag-line form.

Further, instead of the above mentioned groove part 15, the firm attracting through the air intake hole 13 caused by the blockage can be prevented in a way as follows. That is, at least some of the air intake holes 13, 13... can be in communication with each other through a communication path 14 inside the anvil roll 11 in a breathable manner, and at the same time, some of the air intake holes 13, 13... in communication with each other through the communication path 14 can be positioned outside the single-cut film 3 in the CD direction.

For example, in the examples of FIGS. 6 and 7, a communication path 14 that corresponds to the row 13R of air intake holes is formed inside the anvil roll 11 along the CD direction, and the air intake holes 13, 13... that belong to the same row 13R of air intake holes are in communication with each other through the corresponding communication path 14 in a breathable manner. And positions of the air intake holes 13, 13 of each of the rows 13R of air intake holes at both ends in the CD direction are respectively located outside the single-cut film 3 in the width direction as shown in FIG. 7. In such way, each of the air intake holes 13, 13... connected to the communication path 14 takes in outside air through the air intake holes 13, 13 at both ends in the CD direction with relatively small resistance, and thereby the air intake hole 13 is avoided from being in a vacuum sealed state even if the air intake holes 13 other than those at both-ends are completely covered with the single-cut film 3. That is, the peripheral surface 11a is prevented from holding the single-cut film 3 firmly. Note that, a valve can be provided in the communication path 14 for adjusting an air intake amount.

By the way, similar to the groove part 15 described above, such configuration for preventing the vacuum blockage of the communication path 14 or the like is preferably applied especially to the row 13R of air intake holes positioned in the rear part holding area A3r. That is, it is preferable that the communication path 14 is in communication with some of the air intake holes 13, 13... positioned in the rear part holding area A3r in a breathable manner. In this way, the single-cut film 3 slides relatively with respect to the rear part holding area A3r smoothly that should be performed after adhering the leading end part 3e of the single-cut film 3 to the continuous sheet 5aL.

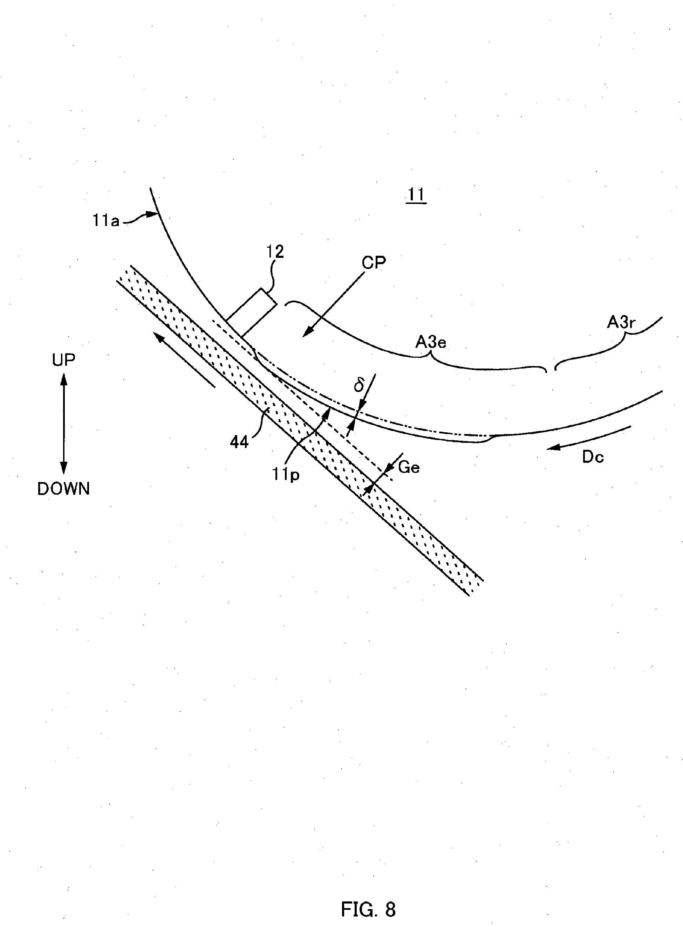

Also it is preferable that, as shown in an enlarged side view of the anvil roll 11 of FIG. 8, the leading end holding area A3e includes a protruded part 11p, and the protruded part 11p protrudes outward in a radial direction of the anvil roll 11 than the remaining area A3r besides the leading end holding area A3e on the peripheral surface 11a. In the illustrated example, as the protruded part 11p, an area having an increased diameter is set in a predetermined area that is from a position of the receiving part 12 to a further upstream side in the circumferential direction Dc (backward side) in the leading end holding area A3e. The amount of increased diameter 5 is, for example, from 0.2 mm to 1.0 mm in radius.

In this way, in the case of adhering the leading end 3e of the single-cut film 3 to the continuous sheet 5aL, the leading end 3e can be pressed against the continuous sheet 5aL by the protruded part 11p of the leading end holding area A3e. As a result, adhesion strength between the leading end part 3e and the continuous sheet 5aL can be increased.

To make the effect of pressing more certain, it is preferable that a space Ge between the protruded part 11p and the belt 44 is set so as to become smaller than the sum of a thickness of the single-cut film 3 and the thickness of the continuous sheet 5aL at the adjacent position CP in which the belt 44 of the suction belt conveyor 43 and the peripheral surface 11a of the anvil roll 11 make the closest approach to each other. By the way, each of the thicknesses can be measured as a distance that appears between a pair of indenters, for example a pair of indenters included in a thickness gage (trade name: PEACOK DIAL THICKNESS GAUGE No.11352), when sandwiching an entire surface of a square sample of 10cm x 10cm under pressure of 3g/cm² in the thickness direction.

Also, in the case where the protruded part 11p is provided in the leading end holding area A3e as described above, the remaining area A3r is relatively distant from the continuous sheet 5aL than the leading end holding area A3e at least for the protruded part 11p. Thus, contact between the continuous sheet 5aL and the peripheral surface 11a can be suppressed while increasing the pressing force applied to the leading end 3e. And in this way, it is possible to inhibit the occurrence of scratch damage on the surface of the continuous sheet 5aL that may be caused by the relative speed difference between the continuous sheet 5aL and the peripheral surface 11a.

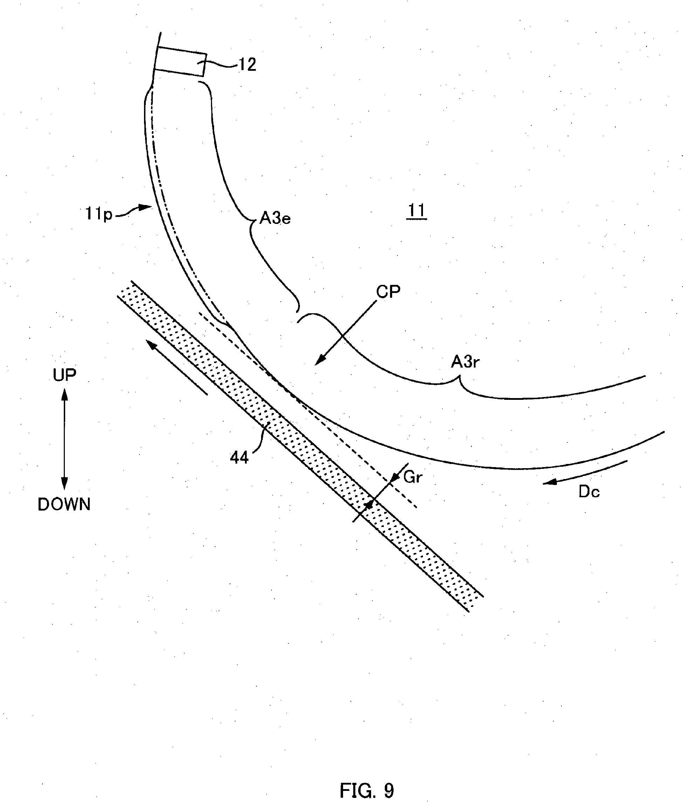

Also, from a viewpoint of suppressing the scratch damage on the continuous sheet 5aL, as shown in FIG. 9, it is preferable that a space Gr between the belt 44 and the remaining area A3r of the peripheral surface 11a at the adjacent position CP is set so as to be larger than the sum of the thickness of the single-cut film 3 and the thickness of the continuous sheet 5aL. In this way, the contact between the remaining area A3r and the continuous sheet 5aL can be avoided and the above mentioned scratch damage on the continuous sheet 5aL can be surely suppressed.

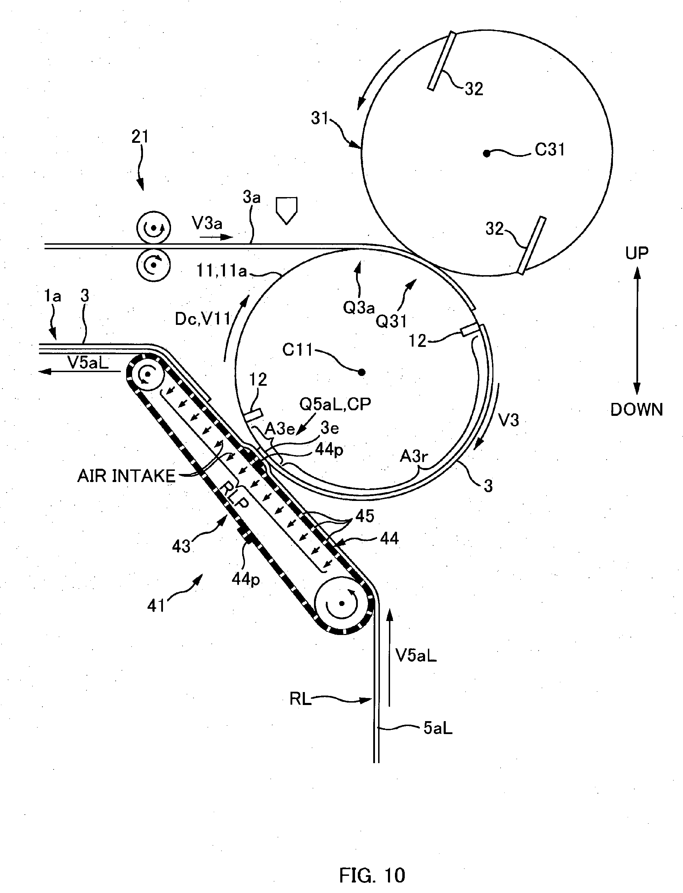

Also, depending on the situation, it is possible to provide a protruded part 44p on the endless belt 44 of the suction belt conveyor 43 instead of the protruded part 11p of the leading end part holding area A3e of FIG. 8, as shown in FIG. 10. That is, while the peripheral surface 11a of the anvil roll 11 is formed by having a uniform radius without including any protruded part 11p in an entire periphery thereof, the protruded part 44p can be formed in a portion which should face the leading end part holding area A3e of the anvil roll 11 in an peripheral surface of the endless belt 44. In the illustrated example, the protruded part 44p is disposed at a predetermined disposition pitch in two positions as an example of the plurality of positions in an orbiting direction of the endless belt 44. This disposition pitch coincides with the adhesion pitch P3L of the single-cut film 3 of a large size. In the protruded part 44p, the air intake hole 45 is provided in the same way as in the other portion of the endless belt 44.

In a case where the configuration of FIG. 10 is adopted, more cost reduction can be achieved than the configuration of FIG. 8 in which the protruded part 11p is provided to the anvil roll 11. This is because the protruded part 44p can be additionally provided to the endless belt 44 at a lower cost than processing and forming the protruded part 11p on the peripheral surface 11a of the anvil roll 11.

By the way, the conveyor 43 of FIG. 10 is used to manufacture intermediate components 1a for large size products and is not necessarily needed to manufacture intermediate components 1a for small size products, however, should there be a case of using the conveyor 43 in manufacturing the intermediate component 1a for small size products, the conveyor 43 will need to be exchanged according to the change in product size from large to small. This is because, the disposition pitch of the protruded part 44p of the endless belt 44 is made to coincide with the adhesion pitch P3 of the single-cut film 3, and the adhesion pitch P3S for small size products and the adhesion pitch P3L for large size products differ from each other. However, the anvil roll 11 does not have to be exchanged in this configuration.

### === Other Embodiments ===

In the above, embodiments according to the present invention were explained. However, the present invention is not limited to the above mentioned embodiments and modifications as described below are possible in so far as such modifications fall within the scope of protection as defined by appended claims.

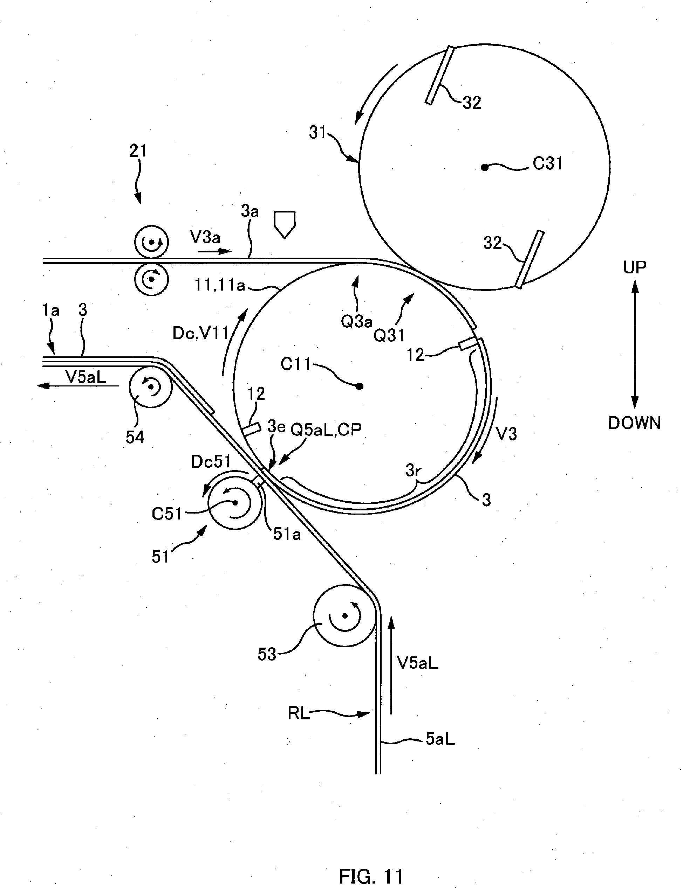

In the above-mentioned embodiments, the method using the air intake mechanism has been exemplified as a method for adhering to the continuous sheet 5aL the leading end 3e of the single-cut film 3 held on the peripheral surface 11a of the anvil roll 11. That is, the leading end 3e of the single-cut film 3 was attracted to the peripheral surface 11a of the anvil roll 11 and adhered to the continuous sheet 5aL by the suction belt conveyor 43 as the air intake mechanism. However, there is no limitation to this and for example, physical pressing can be adopted. More specifically, a hammer roll 51 shown in FIG. 11 can be used.

The hammer roll 51 includes as a main body a roll member disposed in the adjacent position CP by facing the peripheral surface 11a of the anvil roll 11, and is driven to rotate about a rotational axis C51 pointing the CD direction. In a part of a circumferential direction Dc51 thereof, a convex part 51a projecting in a radial direction of the hammer roll 51 is included. And the hammer roll 51 is driven to rotate so that the convex part 51a faces the peripheral surface 11a each time the leading end 3e of the single-cut film 3 held on the peripheral surface 11a of the anvil roll 11 passes the adjacent position CP. In this way, the continuous sheet 5aL is pressed against the leading end 3e of the single-cut film 3 by the convex part 51a and thereby, the leading end 3e is adhered firmly to the continuous sheet 5aL.

By the way, in the case of using the hammer roll 51, the suction belt conveyor 43 can be used as the transport mechanism that forms the transport path RL of the continuous sheet 5aL. However, there is no limitation to this as long as the transport route RL for large size products as mentioned above can be set, and for example, the transport route RL for large size products can be set to run across the continuous sheet 5aL between two pass line rolls 53, 54 as shown in FIG. 11.

In the above-mentioned embodiments, the length L3 (L3S, L3L) of the single-cut film 3 in the circumferential direction Dc in FIG. 3 was not described in detail. However, for example, the length L3 can be set longer than half the length of the disposition pitch P12 of the receiving part 12 on the peripheral surface 11a of the anvil roll 11 in the circumferential direction Dc, and shorter than the disposition pitch P12. And in the case of adopting such setting, the single-cut film 3 is attracted firmly to the peripheral surface 11a of the anvil roll 11 due to the relatively long length thereof, and thereby the single-cut film 3 becomes hard to peel off from the peripheral surface 11a. And the ingenuities exercised on the anvil roll 11 mentioned before exert effects in such a case, and operational advantages thereof (such as preventing the sealed state associated with vacuuming of the air intake hole 13) can be enjoyed sufficiently.

In the above-mentioned embodiments, for the purpose of performing the adhesion of the leading end 3e of the single-cut film 3 to the continuous sheet 5aL not only by suction but also by pressing, an example has been disclosed in which the protruded part 11p is formed in the leading end holding area A3e of the anvil roll 11 as shown in FIG. 8, and the space Ge between the protruded part 11p and the belt 44 of the suction belt conveyor 43 is set to be smaller than the sum of the thickness of the single-cut film 3 and the thickness of the continuous sheet 5aL. However, on the contrary, the space Ge can be set to be larger. And in such case, the leading end 3e of the single-cut film 3 transfers from the leading end holding area A3e to the continuous sheet 5aL by jumping, and in this way, is prevented from being effected by the relative speed difference between the peripheral surface 11a of the anvil roll 11 and the continuous sheet 5aL, and as a result, appearance of wrinkles on the leading end 3e of the single-cut film 3 can be suppressed further reliably.

In the above-mentioned embodiments, the change in size between two sizes such as small and large was mentioned as an example of the change in product size, however, there is no limitation to this, and the change in size between three sizes can be performed by adding medium size, and furthermore, other sizes such as XS and XL can be added too.

In the above-mentioned embodiments, as an example of the transport route RS of the continuous sheet 5aS for small size products that is an example of "the continuous sheet transported at the third speed", as shown in FIG. 4, a path that wraps around the anvil roll 11 at the predetermined wrapping angle θ was exemplified. However, there is no limitation to this, and as same as the transport route RL for large size products of FIG. 5, a transport path that does not wrap around the anvil roll 11 can be 4applied to the continuous sheet 5aS for small size products. In such case, the transport speed V5aS of the continuous sheet 5aS for small size products can be set freely within a range of speed faster than or equal to the peripheral speed V11 of the anvil roll 11 and speed slower than the transport speed V5aL.

In the above-mentioned embodiments, the suction through the air intake holes 13 on the peripheral surface 11a was exemplified as an example of a method of holding the single-cut film 3 or the like on the peripheral surface 11a of the anvil roll 11. However, there is no limitation to this as long as the single-cut film 3 or the like is held in a slidable manner with respect to the peripheral surface 11a.

In the above-mentioned embodiments, the film was mentioned as an example of the single-cut sheet and the first continuous sheet. However, there is no limitation to this as long as it is in a sheet-form and the nonwoven fabric or woven fabric or the like can be used. Also, the nonwoven fabric was mentioned as an example of the second continuous sheet, however, there is no limitation to this as long as it is in a sheet-form, and woven fabric or film or the like can be used.

### Reference Signs List

1 back face sheet, 1a intermediate component (composite of continuous sheet), 3 single-cut film (single-cut sheet, leak-proof film), 3a continuous film (first continuous sheet), 3ae leading end of continuous film, 3e leading end of single-cut film, 3r each rear part, 5 exterior sheet, 5a continuous sheet (second continuous sheet), 5aL continuous sheet (second continuous sheet), 5aS continuous sheet (second continuous sheet), 10 manufacturing apparatus, 11 anvil roll (roll), 11a peripheral surface, 11p protruded part, 12 receiving part (cutter receiving part), 13 air intake hole, 13R row of air intake holes, 14 communication path, 15 groove part, 15e both end parts (a part), 21 continuous film supply mechanism (first supply mechanism), 22a pinch roll, 22b pinch roll, 31 cutter roll (cutter), 31a peripheral surface, 32 flat blade, 41 continuous sheet transport mechanism (second supply mechanism), 43 suction belt conveyor (air intake mechanism), 44 endless belt (belt), 44p protruded part, 45 air intake hole, 47a pulley, 47b pulley, 51 hammer roll, 51a convex part, 53 pass line roll, 54 pass line roll, 81 adhesive applying system, A3 area, A3e leading end holding area, A3r rear part holding area (remaining area), CP adjacent position, Ge space, Gr space, Q3a supply position of continuous film, Q5a supply position of continuous sheet, Q5aL supply position for large size products, Q5aS supply position for small size products, Q31 position of cutter roll, RL transport route for large size products (transport path), RS transport route for small size products (transport path), RLP transport path, C11 rotational axis, C31 rotational axis, C51 rotational axis

## Claims

1. A method of manufacturing a composite of a continuous sheet (1a) for an absorbent article, dividing and producing from a first continuous sheet (3a) single-cut sheets (3) of a predetermined length (L3), and adhering the single-cut sheets to a second continuous sheet (5a) in a continuous direction thereof at a predetermined adhesion pitch (P3), comprising:
holding the first continuous sheet on a peripheral surface (11a) of a roll (11) while sliding, by supplying the first continuous sheet continuously on the peripheral surface of the roll at a first speed (V3a) lower than a peripheral speed (V11) of the roll;
producing the single-cut sheet by dividing the first continuous sheet with a cutter (31) at a time a cutter receiving part (12) provided on the peripheral surface (31a) passes a position of the cutter disposed to face the peripheral surface at a predetermined position (Q31) in a peripheral direction of the roll;
transporting in the peripheral direction and at the peripheral speed the produced single-cut sheet held on the peripheral surface; **characterised by** the steps of
selecting one continuous sheet among a plurality of continuous sheets as the second continuous sheet, the plurality of continuous sheets including a continuous sheet transported at a second speed (V5aL) that is higher than the peripheral speed, and a continuous sheet transported at a third speed (V5aS) that is equal to or higher than the peripheral speed but lower than the second speed; and
adhering the single-cut sheet on the peripheral surface to the continuous sheet by supplying the selected continuous sheet towards the peripheral surface of the roll rotating at the peripheral speed, while coinciding a transport direction of the selected continuous sheet with a rotating direction of the roll.

2. A method of manufacturing a composite of a continuous sheet (1a) for an absorbent article according to claim 1, wherein
in a case the continuous sheet transported at the second speed is selected as the second continuous sheet (5a) in the selecting,
in the adhering,
a leading end (3e) in the peripheral direction of the single-cut sheet (3) is adhered to the second continuous sheet, and thereafter, the single-cut sheet is pulled by the second continuous sheet via the leading end, and while a part of the single-cut sheet held on the peripheral surface slides relatively with respect to the peripheral surface in a travelling direction, the part is gradually peeled off from the peripheral surface to be overlapped and adhered onto the second continuous sheet.

3. A method of manufacturing a composite of a continuous sheet (1a) for an absorbent article according to claim 2, wherein
the single-cut sheet (3) moves together with the peripheral surface at the peripheral speed (V11) until the leading end part (3e) of the single-cut sheet is adhered to the second continuous sheet (5a) , and the single-cut sheet moves together with the second continuous sheet at the second speed while sliding relatively with respect to the peripheral surface in the travelling direction after the leading end part is adhered to the second continuous sheet.

4. A method of manufacturing a composite of a continuous sheet (1a) for an absorbent article according to claim 2 or 3, wherein
the peripheral surface includes a leading end holding area (A3e) that holds the leading end (3ae), and a rear part holding area (A3r) that holds a part rear of the leading end in the peripheral direction, and
a holding force per unit area for holding the single-cut sheet (3) on the peripheral surface is smaller in the rear part holding area than in the leading end holding area.

5. A method of manufacturing a composite of a continuous sheet (1a) for an absorbent article according to any of claims 2 to 4, wherein
the peripheral surface includes the leading end holding area (A3e) that holds the leading end part (3ae) and a remaining area (A3r) that is other than the leading end part holding area, and
the leading end holding area includes a protruded part (44p) protruding outward in a radial direction of the roll beyond the remaining area.

6. A method of manufacturing a composite of a continuous sheet (1a) for an absorbent article according to any of claims 2 to 5, wherein
the peripheral surface includes a width direction that is perpendicular to the peripheral direction,
a plurality of air intake holes (45) are formed on the peripheral surface and the single-cut sheet (3) is attracted and held on the peripheral surface by an air intake through the air intake holes, and
a groove part (15) is formed on the peripheral surface to connect at least some of the air intake holes in a breathable manner, and a part of the groove part is positioned on an outer side of the single-cut sheet in the width direction.

7. A method of manufacturing a composite of a continuous sheet (1a) for an absorbent article according to claim 6, wherein
the peripheral surface includes the leading end holding area (A3e) that holds the leading end (3ae), and the rear part holding area (A3r) that holds the part rear of the leading end in the peripheral direction, and
the groove part (15) is connected to some of the air intake holes (45) positioned in the rear part holding area in a breathable manner.

8. A method of manufacturing a composite of a continuous sheet (1a) for an absorbent article according to any of claims 2 to 7, wherein
the peripheral surface includes a width direction that is perpendicular to the peripheral direction,
a plurality of air intake holes (45) are formed on the peripheral surface and the single-cut sheet (3) is attracted and held on the peripheral surface by the air intake through the air intake holes,
at an inside of the roll, at least some of the air intake holes are in communication with each other through a communication path (14) in a breathable manner, and
some of the air intake holes in communication with each other through the communication path are positioned on an outer side of the single-cut sheet in the width direction.

9. A method of manufacturing a composite of a continuous sheet (1a) for an absorbent article according to claim 8, wherein
the peripheral surface includes the leading end holding area (A3e) that holds the leading end (3ae), and the rear part holding area (A3r) that holds the part rear of the leading end part in the peripheral direction, and
the communication path (14) is in communication with some of the air intake holes (45) positioned in the rear part holding area in a breathable manner.

10. A method of manufacturing a composite of a continuous sheet (1a) for an absorbent article according to any of claims 2 to 9, wherein
the second continuous sheet (5a) has a higher air permeability than the single-cut sheet (3),
a transport path (RL, RS) of the second continuous sheet is in a direction parallel to a tangent direction of the peripheral surface,
an air intake mechanism (43) is provided to an adjacent position (CP) of the transport path closest to the peripheral surface to perform through the second continuous sheet an air intake in a direction that separates the single-cut sheet from the peripheral surface, and
in the adhering,
at a time each portions of the peripheral surface passes the adjacent position, a holding force of each of the portions that holds the single-cut sheet is reduced, and thereby a portion of the single-cut sheet that passes the adjacent position transfers gradually from the peripheral surface to the second continuous sheet.

11. A method of manufacturing a composite of a continuous sheet (1a) for an absorbent article according to claim 10, wherein
the air intake mechanism (43) is a suction belt conveyor that transports the second continuous sheet (5a),
the suction belt conveyor includes a belt (44) having a plurality of air intake holes (45), the belt moving along the transport path while attracting the second continuous sheet by an air intake through the air intake holes, and
in the adhering,
a suction force by the air intake through the air intake holes of the belt acts, through the second continuous sheet, on a portion of the single-cut sheet (3) that is transferred from the peripheral surface to the second continuous sheet.

12. A method of manufacturing a composite of a continuous sheet (1a) for an absorbent article according to claim 11, wherein
the peripheral surface includes the leading end holding area (A3e) that holds the leading end (3ae) and the remaining area (A3r) that is other than the leading end holding area, and
a space between the remaining area and the belt is larger than a sum of a thickness of the single-cut sheet and a thickness of the second continuous sheet.

13. A method of manufacturing a composite of a continuous sheet (1a) for an absorbent article according to any of claims 2 to 4, wherein
the peripheral surface of the roll (11) includes the leading end holding area (A3e) that holds the leading end (3ae), and a remaining area (A3r) that is other than the leading end holding area,
the second continuous sheet (5a) has a higher air permeability than the single-cut sheet (3),
a transport path (RLP) of the second continuous sheet is in a direction parallel to a tangent direction of the peripheral surface,
in an adjacent position (CP) closest the peripheral surface of the transport path, an air intake mechanism (43) is provided to perform an air intake in a direction that separates the single-cut sheet from the peripheral surface through the second continuous sheet,
the air intake mechanism is a suction belt conveyor that transports the second continuous sheet,
the suction belt conveyor includes a belt (44) having a plurality of air intake holes (45), the belt moving along the transport path while attracting the second continuous sheet to a peripheral surface thereof by the air intake through the air intake holes, and
a protruded part (44p) is provided at a portion in the peripheral surface of the belt that should face the leading end holding area of the roll.

14. A method of manufacturing a composite of a continuous sheet (1a) for an absorbent article according to any of claims 1 to 13, wherein
the third speed is same as the peripheral speed,
in the selecting, in a case where the continuous sheet transported at the third speed is selected as the second continuous sheet (5a), the second continuous sheet is transported toward the roll along a transport path (RLP) that wraps around the peripheral surface at a predetermined wrapping angle, and
in the adhering, the single-cut sheet (3) adheres to the second continuous sheet while wrapping around at the wrapping angle.

15. An apparatus (1) for manufacturing a composite of a continuous sheet (1a) for an absorbent article, dividing and producing from a first continuous sheet (3a) single-cut sheets (3) of a predetermined length, and adhering the single-cut sheets to a second continuous sheet (5a) in a continuous direction thereof at a predetermined adhesion pitch (P103), comprising:
a roll (11) that is driven to rotate about a predetermined rotational axis (C11) at a predetermined peripheral speed;
a first supply mechanism (21) that supplies the first continuous sheet to a peripheral surface of the roll;
a cutter (31) disposed to face the peripheral surface at a predetermined position in a peripheral direction of the roll;
a cutter receiving part (12) that is provided on the peripheral surface of the roll and divides the first continuous sheet in cooperation with the cutter; **characterised by**
a second supply mechanism (41) that selects one continuous sheet among a plurality of continuous sheets as the second continuous sheet (5a), the plurality of continuous sheets including a continuous sheet transported at a second speed that is higher than the peripheral speed, and a continuous sheet transported at a third speed that is equal to or higher than the peripheral speed but lower than the second speed, and supplies the selected continuous sheet to the peripheral surface;
wherein the first supply mechanism holds the first continuous sheet on the peripheral surface while sliding, by continuously supplying the first continuous sheet at a first speed lower than the peripheral speed of the roll,
the cutter produces the single-cut sheet by dividing the first continuous sheet in cooperation with the cutter receiving part at a time the cutter receiving part passes a position of the cutter,
the roll transports while holding on the peripheral surface thereof the produced single-cut sheet at the peripheral speed, and
the second supply mechanism supplies the selected continuous sheet towards the peripheral surface of the roll rotating at the peripheral speed and adheres the single-cut sheet on the peripheral surface to the continuous sheet while coinciding a transport direction of the selected continuous sheet with a rotating direction of the roll.

## Patentansprüche

1. Verfahren zur Herstellung eines Verbundstoffs einer kontinuierlichen Schicht (1a) für einen saugfähigen Artikel, Trennung und Erzeugung einer ersten kontinuierlichen Schicht (3a) Einfachschnittschichten (3) eines vorbestimmten Länge (L3), und das Haften der Einfachschnittschichten an eine zweite kontinuierliche Schicht (5a) in einer kontinuierlichen Richtung davon, mit einem vorbestimmten Haftungsgrad (P3), das Folgendes umfasst:
Halten der ersten kontinuierlichen Schicht auf einer peripheren Fläche (11a) einer Walze (11) ist, indem die erste kontinuierliche Schicht kontinuierlich an der peripheren Fläche der Walze mit einer ersten Geschwindigkeit (V3a), die niedriger als eine periphere Geschwindigkeit (V11) der Walze ist, während sie gleitet,
Erzeugung der Einfachschnittschicht, indem die erste kontinuierliche Schicht mit einer Schneidvorrichtung (31) geteilt wird, zu einem Zeitpunkt, an dem die Schneidvorrichtung das Teil (12) empfängt, das an der peripheren Fläche (31 a) bereitgestellt wird und an einer Postion der Schneidvorrichtung vorbeigeht, die so angeordnet ist, dass sie der peripheren Fläche an einer vorbestimmten Position (Q31) gegenübersteht, und zwar in einer peripheren Richtung der Walze,
Transportieren in peripherer Richtung und mit der peripheren Geschwindigkeit, das den produzierte Einfachschnittschicht an der peripheren Fläche hält, **gekennzeichnet durch** die folgenden Schritte:
Wählen einer kontinuierlichen Schicht unter einer Vielzahl von kontinuierlichen Schichten, wenn die zweite kontinuierliche Schicht, die Vielzahl der kontinuierlichen Schichten eine kontinuierliche Schicht beinhalten, die mit einer zweiten Geschwindigkeit (V5aL), die höher ist als die periphere Geschwindigkeit, transportiert wird, und eine kontinuierliche Schicht, die mit einer dritten Geschwindigkeit (V5aS), die gleich oder höher ist als die periphere Geschwindigkeit, aber niedriger als die zweite Geschwindkeit, transportiert wird, und
Haften der Einfachschnittschicht an die periphere Fläche zur kontinuierlichen Schicht, indem die gewählte kontinuierliche Schicht in Richtung der peripheren Fläche einer Walze bereitgestellt wird, die sich mit peripherer Geschwindigkeit dreht, während eine Transportrichtung der gewählten kontinuierlichen Schicht mit der Drehrichtung des Walze übereinstimmt.

2. Verfahren zur Herstellung eines Verbundstoffs einer kontinuierlichen Schicht (1a) für einen saugfähigen Artikel nach Anspruch 1, wobei
in einem Fall, in dem die kontinuierliche Schicht mit der zweiten Geschwindigkeit transportiert wird, die an einer zweiten kontinuierlichen Schicht (5a) bei der Auswahl gewählt wird,
beim Haften,
ein vorderes Ende (3e), das in peripherer Richtung der an der zweiten kontinuierlichen Schicht (3) haftet, und danach wird die an der Einfachschnittschicht von der zweiten kontinuierlichen Schicht über das vorderen Ende gezogen, und während ein Teil der Einfachschnittschicht an der peripheren Fläche gehalten wird, gleitet sie jeweilig hinsichtlich der peripheren Fläche in einer Fahrtrichtung, und das Teil allmählich von der peripheren Fläche abgezogen wird, um dann auf die kontinuierliche Schicht überlappt und gehaftet zu werden.

3. Verfahren zur Herstellung eines Verbundstoffs einer kontinuerlichen Schicht (1a) für einen saugfähigen Artikel nach Anspruch 2, wobei
sich die Einfachschnittschicht (3) mit der peripheren Fläche mit der peripheren Geschwindigkeit (V11) zusammenbewegt, bis das vordere Ende (3e) der Einfachschnittschicht an der zweiten kontinuierlichen Schicht (5a) haftet, und die Einfachschnittschicht sich mit der zweiten kontinuierlichen Schicht zusammenbewegt, mit einer zweiten Geschwindigkeit, während es jeweils im Hinblick auf die periphere Fläche in Transportrichtung gleitet, nachdem das vordere Ende an der zweiten kontinuierlichen Schicht haftet.

4. Verfahren zur Herstellung eines Verbundstoffs einer kontinuierlichen Schicht (1a) für einen saugfähigen Artikel nach Anspruch 2 oder 3, wobei
die periphere Fläche einen Haltebereich (A3e) mit vorderem Ende beinhaltet, das das vordere Ende (3ae) hält, und ein Haltebereich des hinteren Teils (A3r), der ein hinteres Teil des vorderen Endes in peripherer Richtung hält, und
eine Haltekraft pro Einheitsbereich zum Halten der Einfachschnittschicht (3) an der peripheren Fläche, die kleiner als der Haltebereich des hinteren Teils ist, als der Haltebereich des vorderen Ende.

5. Verfahren zur Herstellung eines Verbundstoffs einer kontinuierlichen Schicht (1a) für einen saugfähigen Artikel nach einem der Ansprüche 2 bis 4, wobei
die periphere Fläche den Haltebereich (A3e) des vorderen Endes beinhaltet, der das vordere Endteil (3ae) und einen Restbreich (A3r) hält, der anders ist, als der Haltebereich des vorderen Endteils, und
der Haltebereich des vorderen Endes einen vorstehenden Teil (44p) enthält, der nach außen in radialer Richtung der Walze über den restlichen Bereich hinausragt.

6. Verfahren zur Herstellung eines Verbundstoffs einer kontinuierlichen Schicht (1a) für einen saugfähigen Artikel nach einem der Ansprüche 2 bis 5, wobei
die periphere Fläche eine Breitenrichtung beinhaltet, die senkrecht zur peripheren Richtung ist,
eine Vielzahl von Lufteinlasslöchern (45), die an der peripheren Fläche geformt sind, und die Einfachschnittschicht (3) angezogen wird und an der peripheren Fläche von einem Lufteinlass durch Lufteinlasslöcher gehalten wird, und
das Vertiefungsteil (15) an der peripheren Fläche geformt wird, um wenigstens einige der Lufteinlasslöcher in atembarer Weise anzuschließen, und ein Teil des Vertiefungsteils an einer Außenseite der Einfachschnittschicht in Breitenrichtung positioniert ist.

7. Verfahren zur Herstellung eines Verbundstoffs einer kontinuierlichen Schicht (1a) für einen saugfähigen Artikel nach Anspruch 6, wobei
die periphere Fläche den Haltebereich (A3e) des vorderen Endes beinhaltet, der das vordere Ende (3ae) hält, und den Haltebereich (A3r) des hinteren Teils, der das Teil, das hinter dem vorderen Ende in peripheren Richtung hält, und
das Vertiefungsteil (15) an einigen der Lufteinlasslöcher (45) angeschlossen ist, und die im Haltebereich des hinteren Teils auf eine atembaren Weise positioniert ist.

8. Verfahren zur Herstellung eines Verbundstoffs einer kontinuierlichen Schicht (1a) für einen saugfähigen Artikel nach einem der Ansprüche 2 bis 7, wobei
die periphere Fläche eine Breitenrichtung beinhaltet, die senkrecht zur peripheren Richtung ist,
eine Vielzahl von Lufteinlasslöchern (45), die auf der peripheren Fläche geformt sind, und die Einfachschnittschicht (3) angezogen und auf der peripheren Fläche vom Lufteinlass durch die Lufteinlasslöcher gehalten wird,
an einer Innenseite der Walze wenigsten einige der Lufteinlasslöcher in Verbindung miteinander durch einen Kommunikationspfad (14) auf atembare Weise angebracht sind, und
einige der Lufteinlasslöcher in Verbindung miteinander durch den Kommunikationspfad an einer Außenseite der Einfachschnittschicht in Breitenrichtung positioniert sind.

9. Verfahren zur Herstellung eines Verbundstoffs einer kontinuierlichen Schicht (1a) für einen saugfähigen Artikel nach Anspruch 8, wobei
die peripere Fläche einen Haltebereich (A3e) des vorderen Endes beinhaltet, das das vordere Ende (3ae) hält, und den Haltebereich (A3r) des hinteren Teils, das das hintere Teil des vorderen Endteils in peripherer Richtung hält, und
der Kommunikationspfad (14) in Kommunikation mit einigen der Lufteinlasslöcher (45) sind, der im Haltebereich des hinteren Teils auf atembare Weise positioniert sind.

10. Verfahren zur Herstellung eines Verbundstoffs einer kontinuierlichen Schicht (1 a) für einen saugfähigen Artikel nach einem der Ansprüche 2 bis 9, wobei
die zweite kontinuierliche Schicht (5a) eine höhere Luftdurchlässigkeit als die Einfachschnittschicht (3) hat,
ein Transportpfad (RL, RS) der zweiten kontinuierlichen Schicht in einer Richtung parallel zu einer berührenden Richtung der peripheren Fläche ist,
ein Lufteinlassmeachismus (43), der an einer angrenzenden Position (CP) der Transportpfades, der der peripheren Fläche am nächsten ist, um durch die zweite kontinuerliche Schicht einen Lufteinlass in einer Richtung durchzuführen, die die Einfachschnittschicht von der peripheren Fläche trennt, und
mit der Haftung,
zu einem Zeitpunkt jeder Abschnitt der peripheren Fläche an der angrenzenden Position vorbeigeht, wobei eine Haltekraft von jedem der Abschnitte, die die Einfachschnittschicht halten, reduziert wird, und dabei einen Abschnitt der Einfachschnittschicht, die an der angrenzenden Position vorbeigeht, allmählich von der peripheren Fläche zur zweiten kontinuierlichen Schicht transferiert wird.

11. Verfahren zur Herstellung eines Verbundstoffs einer kontinuierlichen Schicht (1 a) für einen saugfähigen Artikel nach Anspruch 10, wobei
der Lufteinlassmechanismus (43) ein Saugbandförderer ist, der die zweite kontunuierliche Schicht (5a) transportiert,
der Saugbandförderer einen Riemen (44) beinhaltet, der eine Vielzahl von Lufteinlasslöchern (45) hat, wobei das Band sich am Transportpfad entlang bewegt, während es die zweite kontinuierliche Schicht durch einen Lufteinlass durch die Lufteinlasslöcher anzieht, und
mit der Haftung,
eine Saugkraft durch den Lufteinlass durch die Lufteinlasslöcher des Bandes durch die zweite kontinuierliche Schicht auf einen Abschnitt der Einfachschnittschicht (3) wirkt, die von der peripheren Fläche zur zweiten kontinuierlichen Schicht transferiert wird.

12. Verfahren zur Herstellung eines Verbundstoffs einer kontinuierlichen Schicht (1a) für einen saugfähigen Artikel nach Anspruch 11, wobei
die periphere Fläche den Haltebereich (A3e) des vorderen Endes beinhaltet, der das vordere Ende (3ae) und den restlichen Bereich (A3r) hält, der anders als der Haltebereich des vordere Endes ist, und
ein Raum zwischen dem restlichen Bereich und dem Band größer ist, als die Summe einer Dicke der Einfachschnittschicht und einer Dicke der zweiten kontinuierlichen Schicht.

13. Verfahren zur Herstellung eines Verbundstoffs einer kontinuierlichen Schicht (1a) für einen saugfähigen Artikel nach einem der Ansprüche 2 bis 4, wobei
die periphere Fläche der Walze (11), die den Haltebereich (A3e) des vorderen Endes beinhaltet, die das vordere Ende (3ae) hält, und ein restlicher Bereich (A3r), der anders ist, als der Haltebreich des vordereren Endes,
die zweite kontinuerliche Schicht (5a) eine höhere Luftdurchlässigkeit als die Einfachschnittschicht (3) hat,
ein Transportpfad (RLP) der zweiten kontinuierlichen Schicht in einer Richtung parallel zu einer Berührungsrichtung der peripheren Fläche ist,
in einer angrenzenden Position (CP), die der peripheren Fläche des Transportpfads am nächsten ist, einen Lufteinlassmechanismus (43) bereitstellt, um den Lufteinlass in einer Richtung durchzuführen, die die Einfachschnittschicht von der peripheren Fläche bis zur zweiten kontinuierlichen Schicht trennt,
der Lufteinlassmechanismus ein Saugbandförderer ist, der die zweite kontinuierliche Schicht transportiert,
der Saugbandförderer ein Band (44) beinhaltet, das eine Vielzahl von Lufteinlasslöchern (45) hat, wobei sich das Band, das sich am Transportpfad entlang bewegt, während die zweite kontinuierliche Schicht an einer peripheren Fläche davon vom Lufteinlass durch die Lufteinlasslöcher angezogen wird, und
ein vorstehendes Teil (44p) an einem Abschnitt in der peripheren Fläche des Bandes, die dem Haltebereich des vorderen Endes der Walze gegenübersteht, bereitgestellt wird.

14. Verfahren zur Herstellung eines Verbundstoffs einer kontinuierlichen Schicht (1a) für einen saugfähigen Artiel nach einem der Ansprüche 1 bis 13, wobei
die dritte Geschwindigkeit dieselbe ist wie die periphere Geschwindigkeit,
bei der Wahl, in einem Fall, in dem die kontinuierliche Schicht mit der dritten Geschwindigkeit transporiert wird, wird als die zweite kontinuierliche Schicht (5a) gewählt, wobei die zweite kontinuerliche Schicht in Richtung einer Walze an einem Transportpfad (RLP) entlang transportiert wird, die sich um die periphere Fläche in einem vorbestimmten Umhüllungswinkel herumwickelt, und
mit der Haftung, wobei die Einfachschnittschicht (3) an der zweiten kontinuierlichen Schicht haftet, während sie mit dem Umhüllungswinkel herumgewickelt wird.

15. Gerät (1) zur Herstellung eines Verbundstoffs einer kontinuerlichen Schicht (1a) für einen saugfähigen Artikel, der getrennt und von einer ersten kontinuierlichen Schicht (3a), Einfachschnittschichten (3) einer vorbestimmen Länge produziert wird, und die Einfachschnittschichten an einer zweiten kontinuierlichen Schicht (5a) in kontinuierlicher Richtung davon mit einem vorbestimmten Haftgrad (P103) haften, der Folgendes umfasst:
eine Walze (11), die angetrieben wird, um sich um eine vorbestimmte Rotationsachse (C11) mit einer vorbestimmten peripheren Geschwindigkeit zu drehen,
ein erster Versorgungsmechanismus (21), der die erste kontinuierliche Schicht an einer peripheren Fläche der Walze bereitstellt,
eine Schneidvorrichtung (31), der an einer peripheren Fläche in einer vorbestimmten Position in peripherer Richtung der Walze angeordnet ist,
ein Teil zur Aufnahme der Schneidvorrichtung (12), der an der peripheren Fläche einer Walze bereitgestellt wird, und die erste kontinuierliche Schicht in Zusammenarbeit mit der Schneidvorrichtung trennt,
**dadurch gekennzeichnet, dass**
ein zweiter Versorgungsmechanismus (41), der eine kontinuierliche Schicht unter einer Vielzahl von kontinuierlichen Schichten, wie der zweiten kontinuierlichen Schicht (5a) auswählt, wobei die Vielzahl von kontinuierlichen Schichten eine kontinuierliche Schicht beinhaltet, die mit einer zweiten Geschwindigkeit, die höher als die periphere Geschwindigkeit ist, und eine kontinuierliche Schicht mit einer dritten Geschwindigkeit transportiert wird, die gleich oder höher als die periphere Geschwindigkeit ist, aber niedriger als die zweite Geschwindigkeit, und die gewählte kontinuierliche Schicht an der peripheren Fläche bereitstellt,
wobei der erste Versorgungsmechanismus die erste kontinuierliche Schicht an einer peripheren Fläche hält, während sie gleitet, indem die erste kontinuierliche Schicht mit einer ersten Geschwindigkeit versorgt wird, die niedriger als die periphere Geschwindigkeit der Walze ist,
die Schneidvorrichtung die eine Einfachschnittschicht produziert, indem die erste kontinuierliche Schicht in Zusammenarbeit mit dem Empfangsteil der Schneidvorrichtung zu einer Zeit, an der das Empfangsteil der Schneidvorrichtung an einer Position der Schneidvorrichtung vorbeigeht,
die Walzentransporte, während sie die periphere Fläche davon halten, die produzierte Einfachschnittschichte mit der peripheren Geschwindigkeit, und
den zweiten Versorgungsmechanismus, der die gewählte kontinuierliche Schicht in Richtung der peripheren Fläche der Walze versorgt, die sich mit der peripheren Geschwindigkeit dreht und die Einfachschnittschicht an der peripheren Fläche der kontinuierlichen Schicht haftet, während die Transportrichtung der gewählten kontinuierlichen Schicht mit einer Drehrichtung der Walze übereinstimmt.

## Revendications

1. Procédé de fabrication d'un composite d'une feuille continue (1a) pour un article absorbant, en divisant et en produisant des feuilles à coupe simple (3) d'une longueur prédéterminée (L3) à partir d'une première feuille continue (3a) et en faisant adhérer des feuilles à coupe simple à une deuxième feuille continue (5a) dans une direction continue de celle-ci à un pas d'adhésion prédéterminé (P3), comprenant :
maintenir la première feuille continue sur une surface périphérique (11a) d'un rouleau (11) pendant qu'elle glisse, en fournissant la première feuille continue continuellement sur la surface périphérique du rouleau à une première vitesse (V3a) plus basse qu'une vitesse périphérique (V11) du rouleau,
produire la feuille à coupe simple en divisant la première feuille continue avec un couteau (31) au moment où une partie de réception du couteau (12) fournie sur la surface périphérique (31a), passe à une position du couteau disposé pour faire face à la surface périphérique à une position prédéterminée (Q31) dans une direction périphérique du rouleau,
transporter dans la direction périphérique et à la vitesse périphérique, la feuille à simple coupe produite tenue sur la surface périphérique,
**caractérisé par** les étapes consistant à :
sélectionner une feuille continue parmi une pluralité de feuilles continues en tant que deuxième feuille continue, la pluralité de feuilles continues comprenant une feuille continue transportée à une deuxième vitesse (V5aL) qui est plus élevée que la vitesse périphérique, et une feuille continue transportée à une troisième vitesse (V5aS) qui est égale à ou plus élevée que la vitesse périphérique mais plus basse que la deuxième vitesse, et
faire adhérer la feuille à coupe simple sur la surface périphérique à la feuille continue en fournissant la feuille continue sélectionnée vers la surface périphérique du rouleau tournant à la vitesse périphérique, tout en faisant coïncider une direction de transport de la feuille continue sélectionnée avec une direction de rotation du rouleau.

2. Procédé de fabrication d'un composite d'une feuille continue (1a) pour un article absorbant selon la revendication 1, dans lequel :
dans un cas où la feuille continue transportée à la deuxième vitesse est sélectionnée en tant que deuxième feuille continue (5a) à la sélection,
à l'adhésion,
une extrémité avant (3e) dans la direction périphérique de la feuille à coupe simple (3) adhère à la deuxième feuille continue et ensuite la feuille à coupe simple est tirée par la deuxième feuille continue par l'extrémité avant et pendant qu'une partie de la feuille à coupe simple tenue sur la surface périphérique, glisse relativement par rapport à la surface périphérique dans une direction de déplacement, la partie est progressivement détachée de la surface périphérique pour chevaucher et adhérer à la deuxième feuille continue.

3. Procédé de fabrication d'un composite d'une feuille continue (1a) pour un article absorbant selon la revendication 2, dans lequel :
la feuille à coupe simple (3) se déplace avec la surface périphérique à la vitesse périphérique (V11) jusqu'à ce que la partie d'extrémité avant (3e) de la feuille à coupe simple adhère à la deuxième feuille continue (5a) et la feuille à coupe simple se déplace avec la deuxième feuille continue à la deuxième vitesse tout en glissant relativement par rapport à la surface périphérique dans la direction de déplacement après que la partie d'extrémité avant a adhéré à la deuxième feuille continue.

4. Procédé de fabrication d'un composite d'une feuille continue (1a) pour un article absorbant selon la revendication 2 ou 3, dans lequel :
la surface périphérique comprend une zone de maintien d'extrémité avant (A3e) qui maintient l'extrémité avant (3ae) et une zone de maintien d'extrémité arrière (A3r) qui maintient une partie à l'arrière de l'extrémité avant dans la direction périphérique,
et
une force de maintien par unité de surface pour maintenir la feuille à coupe simple (3) sur la surface périphérique est plus petite dans la zone de maintien de la partie arrière que dans la zone de maintien de l'extrémité avant.

5. Procédé de fabrication d'un composite d'une feuille continue (1a) pour un article absorbant selon l'une quelconque des revendications 2 à 4, dans lequel :
la surface périphérique comprend la zone de maintien d'extrémité avant (A3e) qui maintient la partie d'extrémité avant (3ae) et une zone restante (A3r) qui est autre que la zone de maintien de la partie d'extrémité avant, et
la zone de maintien d'extrémité avant comprend une partie en saillie (44p) faisant saillie vers l'extérieur dans une direction radiale du rouleau au-delà de la zone restante.

6. Procédé de fabrication d'un composite d'une feuille continue (1a) pour un article absorbant selon l'une quelconque des revendications 2 à 5, dans lequel :
la surface périphérique comprend une direction de largeur qui est perpendiculaire à la direction périphérique,
une pluralité de trous d'appel d'air (45) sont formés sur la surface périphérique et la feuille à coupe simple (3) est attirée et maintenue sur la surface périphérique par un appel d'air à travers les trous d'appel d'air,
et
une partie de rainure (15) est formée sur la surface périphérique pour se connecter d'une manière respirable à au moins quelques uns des trous d'appel d'air et une partie de la partie de rainure est positionnée sur un côté extérieur de la feuille à coupe simple dans la direction de la largeur.

7. Procédé de fabrication d'un composite d'une feuille continue (1a) pour un article absorbant selon la revendication 6, dans lequel :
la surface périphérique comprend la zone de maintien d'extrémité avant (A3e) qui maintient l'extrémité avant (3ae) et la zone de maintien de la partie arrière (A3r) qui maintient la partie à l'arrière de l'extrémité avant dans la direction périphérique,
et
la partie de rainure (15) est connectée d'une manière respirable à quelques uns des trous d'appel d'air (45) positionnés dans la zone de maintien de la partie arrière.

8. Procédé de fabrication d'un composite d'une feuille continue (1a) pour un article absorbant selon l'une quelconque des revendications 2 à 7, dans lequel :
la surface périphérique comprend une direction de largeur qui est perpendiculaire à la direction périphérique,
une pluralité de trous d'appel d'air (45) sont formés sur la surface périphérique et la feuille à coupe simple (3) est attirée et maintenue sur la surface périphérique par l'appel d'air à travers les trous d'appel d'air,
à l'intérieur du rouleau, au moins quelques uns des trous d'appel d'air sont en communication les uns avec les autres d'une manière respirable à travers un chemin de communication (14), et
quelques uns des trous d'appel d'air en communication les uns avec les autres à travers le chemin de communication, sont positionnés sur un côté extérieur de la feuille à coupe simple dans la direction de la largeur.

9. Procédé de fabrication d'un composite d'une feuille continue (1a) pour un article absorbant selon la revendication 8, dans lequel :
la surface périphérique comprend la zone de maintien d'extrémité avant (A3e) qui maintient l'extrémité avant (3ae) et la zone de maintien de la partie arrière (A3r) qui maintient la partie à l'arrière de la partie d'extrémité avant dans la direction périphérique, et
le chemin de communication (14) est en communication avec quelques uns des trous d'appel d'air (45) positionnés dans la zone de maintien de la partie arrière d'une manière respirable.

10. Procédé de fabrication d'un composite d'une feuille continue (1a) pour un article absorbant selon l'une quelconque des revendications 2 à 9, dans lequel :
la deuxième feuille continue (5a) a une plus grande perméabilité à l'air que la feuille à coupe simple (3),
un chemin de transport (RL, RS) de la deuxième feuille continue est dans une direction parallèle à une direction tangente de la surface périphérique,
un mécanisme d'appel d'air (43) est fourni à une position adjacente (CP) du chemin de transport la plus proche de la surface périphérique pour effectuer à travers la deuxième feuille continue un appel d'air dans une direction qui sépare la feuille à coupe simple de la surface périphérique, et
à l'adhésion,
au moment où chaque partie de la surface périphérique passe à la position adjacente, une force de maintien de chacune des parties qui maintient la feuille à coupe simple est réduite et ainsi une partie de la feuille à coupe simple qui passe à la position adjacente est progressivement transférée de la surface périphérique à la deuxième feuille continue.

11. Procédé de fabrication d'un composite d'une feuille continue (1a) pour un article absorbant selon la revendication 10, dans lequel :
le mécanisme d'appel d'air (43) est une transporteuse à courroie à air aspiré qui transporte la deuxième feuille continue (5a),
la transporteuse à courroie à air aspiré comprend une courroie (44) ayant une pluralité de trous d'appel d'air (45), la courroie se déplaçant le long du chemin de transport tout en attirant la deuxième feuille continue par un appel d'air à travers les trous d'appel d'air, et
à l'adhésion,
une force d'aspiration par l'appel d'air à travers les trous d'appel d'air de la courroie agit, à travers la deuxième feuille continue, sur une partie de la feuille à coupe simple (3) qui est transférée de la surface périphérique à la deuxième feuille continue.

12. Procédé de fabrication d'un composite d'une feuille continue (1a) pour un article absorbant selon la revendication 11, dans lequel :
la surface périphérique comprend la zone de maintien d'extrémité avant (A3e) qui maintient l'extrémité avant (3ae) et la zone restante (A3r) qui est autre que la zone de maintien d'extrémité avant, et
un espace entre la zone restante et la courroie est plus grand qu'une somme d'une épaisseur de la feuille à coupe simple et d'une épaisseur de la deuxième feuille continue.

13. Procédé de fabrication d'un composite d'une feuille continue (1a) pour un article absorbant selon l'une quelconque des revendications 2 à 4, dans lequel :
la surface périphérique du rouleau (11) comprend la zone de maintien d'extrémité avant (A3e) qui maintient l'extrémité avant (3ae) et une zone restante (A3r) qui est autre que la zone de maintien de l'extrémité avant,
la deuxième feuille continue (5a) a une plus grande perméabilité à l'air que la feuille à coupe simple (3),
un chemin de transport (RLP) de la deuxième feuille continue est dans une direction parallèle à une direction tangente de la surface périphérique,
à une position adjacente (CP) la plus proche de la surface périphérique du chemin de transport, un mécanisme d'appel d'air (43) est fourni pour effectuer un appel d'air dans une direction qui sépare la feuille à coupe simple de la surface périphérique à travers la deuxième feuille continue,
le mécanisme d'appel d'air est une transporteuse à courroie à air aspiré qui transporte la deuxième feuille continue,
la transporteuse à courroie à air aspiré comprend une courroie (44) ayant une pluralité de trous d'appel d'air (45), la courroie se déplaçant le long du chemin de transport tout en attirant la deuxième feuille continue vers une surface périphérique de celle-ci par l'appel d'air à travers les trous d'appel d'air, et
une partie en saillie (44p) est fournie à une partie de la surface périphérique de la courroie qui devrait faire face à la zone de maintien d'extrémité avant du rouleau.

14. Procédé de fabrication d'un composite d'une feuille continue (1a) pour un article absorbant selon l'une quelconque des revendications 1 à 13, dans lequel :
la troisième vitesse est la même que la vitesse périphérique,
à la sélection, dans un cas où la feuille continue transportée à la troisième vitesse est sélectionnée en tant que deuxième feuille continue (5a), la deuxième feuille continue est transportée vers le rouleau le long d'un chemin de transport (RLP) qui l'enroule autour de la surface périphérique à un angle d'enroulement prédéterminé, et
à l'adhésion, la feuille à coupe simple (3) adhère à la deuxième feuille continue pendant qu'elle s'enroule à l'angle d'enroulement.

15. Appareil (1) de fabrication d'un composite d'une feuille continue (1a) pour un article absorbant, en divisant et en produisant des feuilles à coupe simple (3) d'une longueur prédéterminée à partir d'une première feuille continue (3a) et en faisant adhérer les feuilles à coupe simple à une deuxième feuille continue (5a) dans une direction continue de celle-ci à un pas d'adhésion prédéterminé (P103), comprenant :
un rouleau (11) qui est entraîné pour tourner autour d'un axe de rotation prédéterminé (C11) à une vitesse périphérique prédéterminée,
un premier mécanisme d'alimentation (21) qui fournit la première feuille continue à une surface périphérique du rouleau,
un couteau (31) disposé pour faire face à la surface périphérique à une position prédéterminée dans une direction périphérique du rouleau,
une partie de réception du couteau (12) qui est fournie sur la surface périphérique du rouleau et divise la première feuille continue en coopération avec le couteau, **caractérisé en ce que** :
un deuxième mécanisme d'alimentation (41) qui sélectionne une feuille continue parmi une pluralité de feuilles continues en tant que deuxième feuille continue (5a), la pluralité de feuilles continues comprenant une feuille continue transportée à une deuxième vitesse qui est plus élevée que la vitesse périphérique et une feuille continue transportée à une troisième vitesse qui est égale à ou plus élevée que la vitesse périphérique mais plus basse que la deuxième vitesse, et fournit la feuille continue sélectionnée à la surface périphérique,
dans lequel le premier mécanisme d'alimentation maintient la première feuille continue sur la surface périphérique pendant qu'elle glisse, en fournissant continuellement la première feuille continue à une première vitesse plus basse que la vitesse périphérique du rouleau,
le couteau produit la feuille à coupe simple en divisant la première feuille continue en coopération avec la partie de réception du couteau au moment où la partie de réception du couteau passe à une position du couteau,
le rouleau transporte la feuille à coupe simple produite à la vitesse périphérique tout en la maintenant sur la surface périphérique de celui-ci, et
le deuxième mécanisme d'alimentation fournit la feuille continue sélectionnée vers la surface périphérique du rouleau tournant à la vitesse périphérique et fait adhérer la feuille à coupe simple sur la surface périphérique à la feuille continue tout en faisant coïncider une direction de transport de la feuille continue sélectionnée avec une direction de rotation du rouleau.
